# EUROPEAN PATENT SPECIFICATION

(11) **EP 4 125 572 B1**
(45) Date of publication and mention of the grant of the patent: **29.04.2026**
(21) Application number: 21717545.4
(22) Date of filing: 31.03.2021
(51) Int. Cl.: A61B 5/08, A61B 5/11, A61B 5/00, A61M 16/00

(54) **SYSTEM FOR MAPPING AN AIRWAY OBSTRUCTION**
SYSTEM ZUR ABBILDUNG EINER ATEMWEGSOBSTRUKTION
SYSTÈME POUR CARTOGRAPHIER UNE OBSTRUCTION DES VOIES RESPIRATOIRES

(30) Priority: 31.03.2020 US 202063002790 P; 03.06.2020 US 202062704944 P
(43) Date of publication of application: 08.02.2023
(73) Proprietor: ResMed Sensor Technologies Limited, Sandyford D18 T6T7 Dublin (IE)
(72) Inventor: LYON, Graeme, Alexander, Dublin, D18 T6T7 (IE)
(74) Representative: Mathys & Squire
(86) International application number: PCT/IB2021/052678
(87) International publication number: WO 2021/198941

(56) References cited:
- WO-A1-2010/091462
- US-A1- 2005 005 935
- US-A1- 2006 037 615
- US-A1- 2019 388 024
- GELARDI MATTEO ET AL: "Acoustic pharyngometry: clinical and instrumental correlations in sleep disorders", BRAZILIAN JOURNAL OF OTORHINOLARYNGOLOGY, vol. 73, no. 2, 28 March 2007 (2007-03-28), pages 257 - 265, XP055808688, ISSN: 1808-8694, Retrieved from the Internet <URL:http://dx.doi.org/10.1016/S1808-8694(15)31075-2> DOI: 10.1016/S1808-8694(15)31075-2

## Description

### CROSS-REFERENCE TO RELATED APPLICATIONS

This application claims the benefit of, and priority to, U.S. Provisional Patent Application Serial No. 63/002,790, filed on March 31, 2020, titled "System and Method for Mapping an Airway Obstruction," and the benefit of, and priority to, U.S. Provisional Patent Application Serial No. 62/704,944, filed on June 3, 2020, titled "System and Method for Mapping an Airway Obstruction".

### TECHNICAL FIELD

The present disclosure relates generally to systems and methods for determining an apnea obstruction, and more particularly, to systems and methods for estimating physical and temporal characteristics of the apnea obstruction using an acoustic reflection.

### BACKGROUND

Many individuals suffer from sleep-related and/or respiratory-related disorders such as, for example, Periodic Limb Movement Disorder (PLMD), Restless Leg Syndrome (RLS), Sleep-Disordered Breathing (SDB) such as Obstructive Sleep Apnea (OSA), Central Sleep Apnea (CSA) and other types of apneas such as hypopnea, hyperpnea, and hypercapnia, Cheyne-Stokes Respiration (CSR), respiratory insufficiency, Obesity Hyperventilation Syndrome (OHS), Chronic Obstructive Pulmonary Disease (COPD), Neuromuscular Disease (NMD), and chest wall disorders. These disorders are often treated using respiratory therapy systems. Obstructive Sleep Apnea (OSA) is a form of Sleep Disordered Breathing (SDB), and is characterized by events including occlusion or obstruction of the upper air passage during sleep which may result from a combination of an abnormally small upper airway and the normal loss of muscle tone in the region of the tongue, soft palate and posterior oropharyngeal wall. More generally, an apnea generally refers to the cessation of breathing caused by blockage of the air (Obstructive Sleep Apnea) or the stopping of the breathing function (Central Sleep Apnea). Mixed apneas are apnea events that are combination of both obstructive and central sleep apnea symptoms, and such events typically begin as a central apneas and end as obstructive apneas. Hypopnea is generally characterized by slow or shallow breathing caused by a narrowed airway, as opposed to a blocked airway. Cheyne-Stokes Respiration (CSR) is another form of Sleep Disordered Breathing. CSR is a disorder of a patient's respiratory controller in which there are rhythmic alternating periods of waxing and waning ventilation known as CSR cycles. CSR is characterized by repetitive de-oxygenation and re-oxygenation of the arterial blood US 2006/037615 A1 relates to breathing events.

An occlusion or obstruction may be characterized by a partial or full collapse of the upper airway during sleep. This physical site of the airway obstruction is an important consideration in understanding a patient's disease state and the optimal solution for their disorder. However, the nature of airway collapse is typically difficult to measure without dedicated or invasive hardware, especially during standard PAP therapy in a home setting.

One such method in existence for example is DISE (Drug-induced sedation endoscopy). This sedates a patient to simulate natural sleep and examines the airway optically though an endoscope. A classification of the airway obstruction is then made manually by an expert using the VOTE system (velum, oropharynx, tongue base and epiglottis). Other methods involve affixing pressure sensors within the upper airway or using imaging techniques such as fluoroscopy, CT, MRI and radiography. Some automated methods that analyze snoring audio or airflow signals have been reported; however, these methods are less direct, may not work during PAP therapy, and may only be able to infer the site of obstruction during partial airway collapse (hypopnea).

The present disclosure is directed to solving these and other problems, including problems associated with determining physical and temporal characteristics of an apnea obstruction.

### SUMMARY

The current invention is defined in the claims. According to some implementations of the present disclosure, a method includes emitting an acoustic signal into an airway of a user. The method further includes detecting an acoustic reflection of the acoustic signal caused by one or more physical features within the airway of the user. The method also includes analyzing acoustic data associated with the acoustic reflection. The method also further includes characterizing and/or determining an occurrence of a physical obstruction in the airway of the user. Characterizing and/or determining the apnea obstruction is based, at least in part, on the analyzed acoustic data. The characterization is indicative of an apnea event or a hypopnea event in the user, wherein the apnea event comprises an obstructive apnea event, a central apnea event or a mixed apnea event, and further distinguishes between the occurrence of the obstructive apnea event, the central apnea event, the mixed apnea event, or the hypopnea event in the user.

According to other implementations of the present disclosure, a system includes an acoustic device for emitting an acoustic signal and a memory device storing machine-readable instructions. The system further includes a control system including one or more processors communicatively coupled to the acoustic device. The control system is configured to execute the machine-readable instructions to detect an acoustic reflection of the acoustic signal emitted by the acoustic device into the airway of the user. The acoustic reflection is caused by a one or more physical features within the airway of the user. Acoustic data associated with the acoustic reflection is analyzed, and an occurrence of the physical obstruction is characterized in the airway of the user. The characterization is determined based, at least in part, on the analyzed acoustic data. The characterization is indicative of an apnea event or a hypopnea event in the user, wherein the apnea event comprises an obstructive apnea event, a central apnea event or a mixed apnea event, and further distinguishes between the occurrence of the obstructive apnea event, the central apnea event, the mixed apnea event, or the hypopnea event in the user.

According to yet other implementations of the present disclosure, a system includes a respiratory therapy system and an acoustic device associated with the respiratory therapy system. The acoustic device is configured to emit an acoustic signal into an airway of a user. The system further includes a memory device storing machine-readable instructions and a control system including one or more processors communicatively coupled to the acoustic device. The control system is configured to execute the machine-readable instructions to supply pressurized air, via the respiratory system, to the airway of the user during a sleep session. The instructions are further executed to receive flow data associated with the pressurized air, and to determine, based on the flow data, occurrence of an apnea event or a hypopnea event. The instructions are also executed to receive an acoustic reflection based on one or more physical features within the airway of the user. The instructions are also further executed to analyze acoustic data associated with the acoustic reflection, and to characterize physical and/or temporal characteristics of the physical obstruction based, at least in part, on the analyzed acoustic data. The characterization is indicative of an apnea event or a hypopnea event in the user, wherein the apnea event comprises an obstructive apnea event, a central apnea event or a mixed apnea event, and further distinguishes between the occurrence of the obstructive apnea event, the central apnea event, the mixed apnea event, or the hypopnea event in the user.

The above summary is not intended to represent each embodiment or every aspect of the present disclosure. Rather, the foregoing summary merely provides an example of some of the novel aspects and features set forth herein. The above features and advantages, and other features and advantages of the present disclosure, will be readily apparent from the following detailed description of representative embodiments and modes for carrying out the present invention, when taken in connection with the accompanying drawings and the appended claims. Additional aspects of the disclosure will be apparent to those of ordinary skill in the art in view of the detailed description of various embodiments, which is made with reference to the drawings, a brief description of which is provided below.

### BRIEF DESCRIPTION OF THE DRAWINGS

The disclosure, and its advantages and drawings, will be better understood from the following description of exemplary embodiments together with reference to the accompanying drawings. These drawings depict only exemplary embodiments, and are therefore not to be considered as limitations on the scope of the various embodiments or claims.
FIG. 1 is a functional block diagram of a system for determining an occurrence of an apnea obstruction, according to some implementations of the present disclosure.
FIG. 2A is a perspective view of at least a portion of the system of FIG. 1, a user wearing a full face mask, and a bed partner, according to some implementations of the present disclosure.
FIG. 2B is a perspective view of at least a portion of the system of FIG. 1, a user wearing a nasal mask, and a bed partner, according to some implementations of the present disclosure.
FIG. 3A is a diagram that illustrates an overview of a respiratory system of a user.
FIG. 3B is a diagram that illustrates nasal and mouth airways of the user of FIG. 3A.
FIG. 4 is an exemplary cepstrum plot representing a physical obstruction within a user airway, according to some implementations of the present disclosure.
FIG. 5 is a side view illustration of a system for determining the physical obstruction of FIG. 4, according to some implementations of the present disclosure.
FIG. 6 is a side view illustration of a partial collapsed state of a user airway.
FIG. 7 is a side view illustration of a fully collapsed state of a user airway.
FIG. 8 is a front view illustration of a mobile phone displaying options based on a physical obstruction, according to some implementations of the present disclosure.
FIG. 9 is a front view illustration of a mobile phone displaying further options based on a physical obstruction, according to some implementations of the present disclosure.
FIG. 10 illustrates a system including a mobile phone, a respiratory device, and a mask for characterizing a physical obstruction in a user airway, according to some implementations of the present disclosure.
FIG. 11 illustrates a system including a mobile phone and an acoustic device for characterizing a physical obstruction in a user airway, according to some implementations of the present disclosure.
FIG. 12 is a display illustrating an image representative of a full throat collapse, according to some implementations of the present disclosure.
FIG. 13 is a display illustrating an image representative of a partial throat collapse, according to some implementations of the present disclosure.
FIG. 14 is a display illustrating an image representative of a time period for monitoring an apnea event, according to some implementations of the present disclosure.
FIG. 15 is a display illustrating an image representative of a first set of settings for characterizing a physical obstruction during an apnea event, according to some implementations of the present disclosure.
FIG. 16 is a display illustrating an image representative of a second set of settings for characterizing a physical obstruction during an apnea event, according to some implementations of the present disclosure.
FIG. 17 is a display illustrating an image representative of a physical obstruction, according to some implementations of the present disclosure.

While the invention is susceptible to various modifications and alternative forms, specific implementations have been shown by way of example in the drawings and will be described in further detail herein. It should be understood, however, that the invention is not intended to be limited to the particular forms disclosed. Rather, the invention is to cover all modifications, equivalents, and alternatives falling within the scope of the invention as defined by the appended claims.

### DETAILED DESCRIPTION

Various embodiments are described with reference to the attached figures, where like reference numerals are used throughout the figures to designate similar or equivalent elements. The figures are not drawn to scale and are provided merely to illustrate the instant invention. Several aspects of the invention are described below with reference to example applications for illustration. It should be understood that numerous specific details, relationships, and methods are set forth to provide a full understanding of the invention. One having ordinary skill in the relevant art, however, will readily recognize that the invention can be practiced without one or more of the specific details, or with other methods. In other instances, well-known structures or operations are not shown in detail to avoid obscuring the invention. The various embodiments are not limited by the illustrated ordering of acts or events, as some acts may occur in different orders and/or concurrently with other acts or events. Furthermore, not all illustrated acts or events are required to implement a methodology in accordance with the present invention.

Elements and limitations that are disclosed, for example, in the Abstract, Summary, and Detailed Description sections, but not explicitly set forth in the claims, should not be incorporated into the claims, singly, or collectively, by implication, inference, or otherwise. For purposes of the present detailed description, unless specifically disclaimed, the singular includes the plural and vice versa. The word "including" means "including without limitation." Moreover, words of approximation, such as "about," "almost," "substantially," "approximately," "generally," and the like, can be used herein to mean "at," "near," or "nearly at," or "within 3-5% of," or "within acceptable manufacturing tolerances," or any logical combination thereof, for example.

Generally, the present disclosure describes a system and method in which an acoustic reflection is used to non-invasively characterize an airway obstruction. The airway obstruction may be a physical obstruction (e.g., such as a tongue base of the user) in the airway that is deemed to be an apnea obstruction because it causes and occurs during an apnea event or a hypopnea obstruction because it causes and occurs during a hypopnea event.

A partially or fully collapse airway presents a change in acoustic impedance that produces a reflection when provided with acoustic stimulus, i.e., an acoustic signal emitted into an airway of a user. The site and/or nature of the airway collapse is estimated by analyzing reflections that occur within the airway of the user (i.e., beyond a mouth or mask of the user). The obstruction site is characterized based, at least in part, on a distance travelled into the airway and/or signal morphology of the reflected signal.

In addition to determining obstructive apnea or hypopnea events, the disclosed system and method can characterize a central apnea (non-obstructive) event, and distinguish such events from obstructive apnea or hypopnea events. During a central apnea event, a user's airway remains open (i.e., is not obstructed), but the user stops breathing. Thus, there is an absence of a physical obstruction in the airway of the user. The central apnea is determined based on breathing (respiration) data indicating that he user has stopped breathing. The breathing data, which is indicative of the user's breathing pattern, is obtained, for example, from a respiratory therapy system (e.g., a positive airway pressure (PAP) system), from a motion sensor suitable to detect respiration-related movement such as a sonar or radar sensor, or from a passive acoustic sensor (e.g., a microphone). Various methods may be employed to extract a breathing signal from data generated by a passive acoustic sensor, such as a microphone. typically in the vicinity of the user. For example, the envelope of the time domain signal may be measured using an appropriately sized window. There are many envelope detection types such as, for example, root mean square (RMS), maximum, minimum, peak, Hilbert transform, etc. Additionally or alternatively, the mean, median and/or standard deviation of the time domain signal or the absolute of the time domain signal may be analyzed. Further still, the amplitude over time of one or more frequency bands of the passive acoustic signal may be measured.

Thus, an obstructive apnea or hypopnea event results in a pause in breathing or a reduction in ventilation due to a partial or full obstruction of the airway of the user. Respiratory effort is made on the part of the user, but airflow in the airway is impeded. In contrast, a central apnea event results in a pause in breathing without an associated respiratory effort. In other words, the brain does not send a signal to breath, but the airway remains open and unobstructed.

Conventionally, and in general terms, an apnea would be detected via the flow signal by determining if a pause in breathing has occurred, e.g., a period of time such as, 8 seconds, elapsing with no flow signal indicative of breathing. Similarly, hypopneas would be characterised by a predetermined reduction in airflow over a period of time, e.g., 30% reduction in flow amplitude lasting >6 seconds.

Currently, to discriminate obstructive apnea events from central apnea events, the respiratory device employs a technique called forced oscillation (FOT) which investigates the airway patency by applying an oscillating 4 Hertz (Hz) pressure waveform of 1 cmH₂O and determining if a response is observed in the flow. If there is a 4 Hz oscillation observed in flow then the airway is deemed open and the event would be classified as central, if no 4 Hz component is present in the flow signal then the event would be deemed obstructive. One downside of FOT is that these pressure oscillations may cause discomfort to the patient or even awaken them.

**In** accordance with the present disclosure, the acoustic data can achieve both (a) the detection of apneas/hypopneas events, and (b) discrimination of central from obstructive events. As further disclosed below, an obstruction in the airway may be characterized using acoustic data. The characterization may indicate absence of an airway obstruction indicative that a central event is occurring during periods where a breathing pause has been detected (via a flow and/or pressure signal from a respiratory therapy system, passive acoustic data generated by a passive acoustic sensor, a motion sensor such as a radar or sonar sensor, or otherwise). One benefit of this method is that it avoids the potential discomfort associated with the FOT method.

Furthermore, also in accordance with the present disclosure, a user's breathing is clearly present in the acoustic data associated with the acoustic reflection and a breathing signal can be extracted using a variety of time or frequency methods. Consequently, apneas are detected by applying rules as described above for flow and/or pressure signals, e.g., the absence of breathing for >X seconds. Similarly, the breathing amplitude may be extracted from the acoustic data and used to determine a reduction in breathing amplitude, and hence detect hypopneas or confirm the detection of hypopneas.

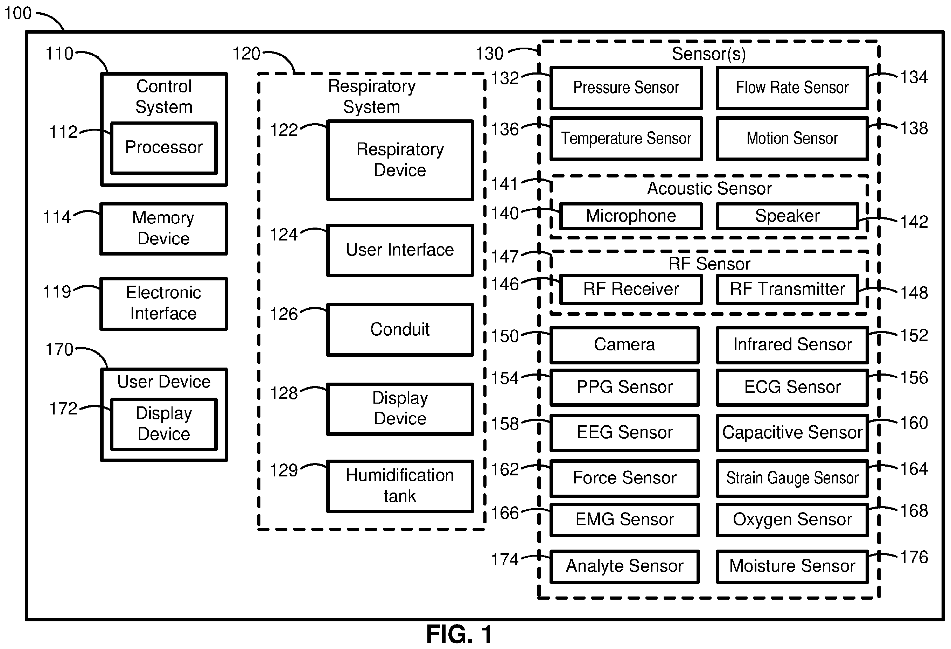

Referring to FIG. 1, a system 100, according to some implementations of the present disclosure, is illustrated. The system 100 includes at least a control system 110, a memory device 114, and an acoustic device, according to some implementations. The acoustic device, according to some exemplary implementations, is in the form of a microphone 140 and/or a speaker 142. In some implementations, the acoustic device includes a passive acoustic sensor, such as microphone 140. The passive acoustic sensor is suitable for generating passive acoustic data associated with the breathing of a user. The system 100 further includes, according some other implementations, an electronic interface 119, one or more sensors 130 (which optionally include the microphone 140 and/or the speaker 142), and one or more user devices 170. In some further implementations, the system 100 includes a respiratory therapy system 120.

The control system 110 includes one or more processors 112 (hereinafter, processor 112). The control system 110 is generally used to control (e.g., actuate) various components of the system 100 and/or analyze data obtained and/or generated by the components of the system 100. The processor 112 can be a general or special purpose processor or microprocessor. While one processor 112 is shown in FIG. 1, the control system 110 can include any suitable number of processors (e.g., one processor, two processors, five processors, ten processors, etc.) that can be in a single housing, or located remotely from each other. The control system 110 can be coupled to and/or positioned within, for example, a housing of the user device 170, and/or within a housing of one or more of the sensors 130. The control system 110 can be centralized (within one such housing) or decentralized (within two or more of such housings, which are physically distinct). In such implementations including two or more housings containing the control system 110, such housings can be located proximately and/or remotely from each other.

The memory device 114 stores machine-readable instructions that are executable by the processor 112 of the control system 110. The memory device 114 can be any suitable computer readable storage device or media, such as, for example, a random or serial access memory device, a hard drive, a solid state drive, a flash memory device, etc. While one memory device 114 is shown in FIG. 1, the system 100 can include any suitable number of memory devices 114 (e.g., one memory device, two memory devices, five memory devices, ten memory devices, etc.). The memory device 114 can be coupled to and/or positioned within a housing of the respiratory device 122, within a housing of the user device 170, within a housing of one or more of the sensors 130, or a combination thereof. Like the control system 110, the memory device 114 can be centralized (within one such housing) or decentralized (within two or more of such housings, which are physically distinct).

The electronic interface 119 is configured to receive data (e.g., physiological data) from the one or more sensors 130 such that the data can be stored in the memory device 114 and/or analyzed by the processor 112 of the control system 110. The electronic interface 119 can communicate with the one or more sensors 130 using a wired connection or a wireless connection (e.g., using an RF communication protocol, a Wi-Fi communication protocol, a Bluetooth communication protocol, over a cellular network, etc.). The electronic interface 119 can include an antenna, a receiver (e.g., an RF receiver), a transmitter (e.g., an RF transmitter), a transceiver, or a combination thereof. The electronic interface 119 can also include one more processors and/or one more memory devices that are the same as, or similar to, the processor 112 and the memory device 114 described herein. In some implementations, the electronic interface 119 is coupled to or integrated in the user device 170. In other implementations, the electronic interface 119 is coupled to or integrated (e.g., in a housing) with the control system 110 and/or the memory device 114.

As noted above, in some implementations, the system 100 can include a respiratory therapy system 120. The respiratory therapy system 120 can include a respiratory pressure therapy (RPT) device 122 (referred to herein as respiratory device 122), a user interface 124 (also referred to as a mask), a conduit 126 (also referred to as a tube or an air circuit), a display device 128, a humidification tank 129, a receptacle 180, or a combination thereof. In some implementations, the control system 110, the memory device 114, the display device 128, one or more of the sensors 130, and the humidification tank 129 are part of the respiratory device 122. Respiratory pressure therapy refers to the application of a supply of air to an entrance to a user's airways at a controlled target pressure that is nominally positive with respect to atmosphere throughout the user's breathing cycle (e.g., in contrast to negative pressure therapies such as the tank ventilator or cuirass). The respiratory therapy system 120 is generally used to treat individuals suffering from one or more sleep-related respiratory disorders (e.g., obstructive sleep apnea, central sleep apnea, or mixed sleep apnea).

The respiratory device 122 is generally used to generate pressurized air that is delivered to a user (e.g., using one or more motors that drive one or more compressors). In some implementations, the respiratory device 122 generates continuous constant air pressure that is delivered to the user. In other implementations, the respiratory device 122 generates two or more predetermined pressures (e.g., a first predetermined air pressure and a second predetermined air pressure). In still other implementations, the respiratory device 122 is configured to generate a variety of different air pressures within a predetermined range. For example, the respiratory device 122 can deliver at least about 6 cmH₂O, at least about 10 cmH₂O, at least about 20 cmH₂O, between about 6 cmH₂O and about 10 cmH₂O, between about 7 cmH₂O and about 12 cmH₂O, etc. The respiratory device 122 can also deliver pressurized air at a predetermined flow rate between, for example, about -20 L/min and about 150 L/min, while maintaining a positive pressure (relative to the ambient pressure).

The user interface 124 engages a portion of the user's face and delivers pressurized air from the respiratory device 122 to the user's airway to aid in preventing the airway from narrowing and/or collapsing during sleep. Generally, the user interface 124 engages the user's face such that the pressurized air is delivered to the user's airway via the user's mouth, the user's nose, or both the user's mouth and nose. Together, the respiratory device 122, the user interface 124, and the conduit 126 form an air pathway fluidly coupled with an airway of the user. The pressurized air also increases the user's oxygen intake during sleep.

Depending upon the therapy to be applied, the user interface 124 may form a seal, for example, with a region or portion of the user's face, to facilitate the delivery of gas at a pressure at sufficient variance with ambient pressure to effect therapy, for example, at a positive pressure of about 10 cmH₂O relative to ambient pressure. For other forms of therapy, such as the delivery of oxygen, the user interface may not include a seal sufficient to facilitate delivery to the airways of a supply of gas at a positive pressure of about 10 cmH₂O.

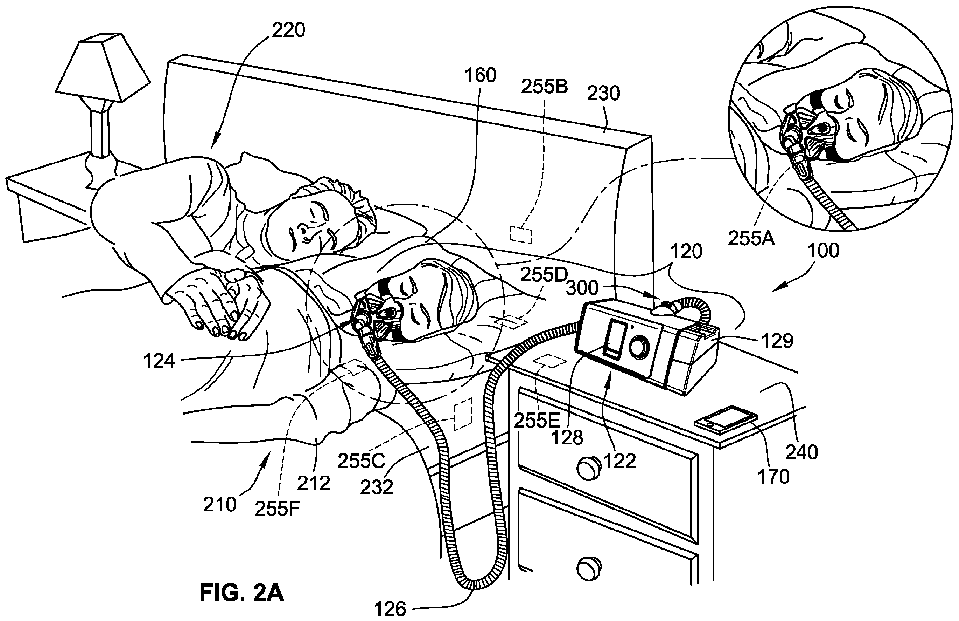

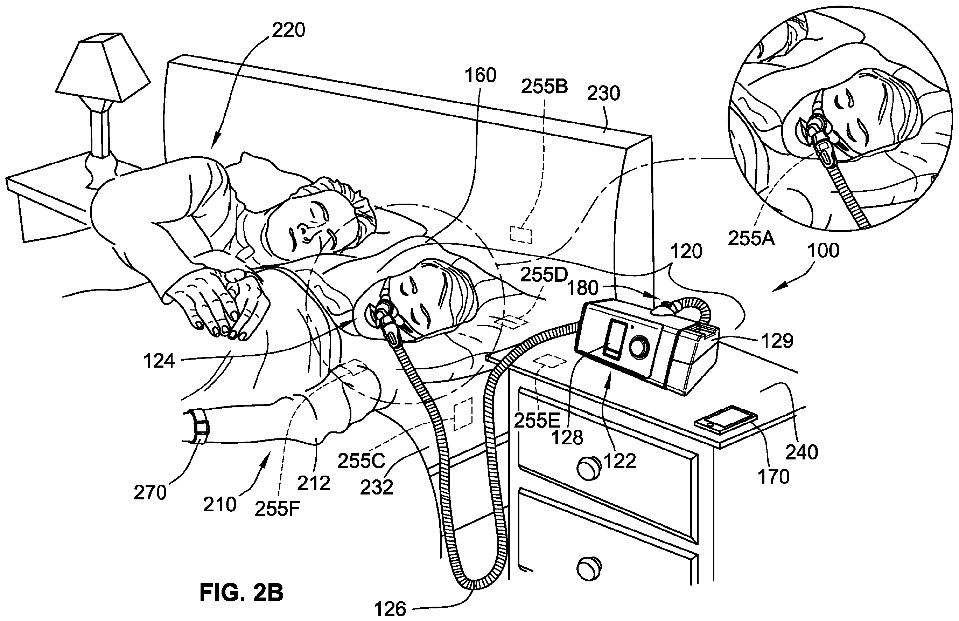

As shown in FIG. 2A, in some implementations, the user interface 124 is a facial mask (e.g., a full face mask) that covers the nose and mouth of the user. Alternatively, as shown in FIG. 2B, the user interface 124 is a nasal mask that provides air to the nose of the user or a nasal pillow mask that delivers air directly to the nostrils of the user. The user interface 124 can include a plurality of straps (e.g., including hook and loop fasteners) for positioning and/or stabilizing the user interface on a portion of the user (e.g., the face) and a conformal cushion (e.g., silicone, plastic, foam, etc.) that aids in providing an air-tight seal between the user interface 124 and the user. The user interface 124 can also include one or more vents for permitting the escape of carbon dioxide and other gases exhaled by the user 210. In other implementations, the user interface 124 includes a mouthpiece (e.g., a night guard mouthpiece molded to conform to the user's teeth, a mandibular repositioning device, etc.).

The conduit 126 (also referred to as an air circuit or tube) allows the flow of air between two components of a respiratory therapy system 120, such as the respiratory device 122 and the user interface 124. In some implementations, there can be separate limbs of the conduit for inhalation and exhalation. In other implementations, a single limb conduit is used for both inhalation and exhalation.

One or more of the respiratory device 122, the user interface 124, the conduit 126, the display device 128, and the humidification tank 129 can contain one or more sensors (e.g., a pressure sensor, a flow rate sensor, or more generally any of the other sensors 130 described herein). These one or more sensors can be used, for example, to measure the air pressure and/or flow rate of pressurized air supplied by the respiratory device 122.

The display device 128 is generally used to display image(s) including still images, video images, or both and/or information regarding the respiratory device 122. For example, the display device 128 can provide information regarding the status of the respiratory device 122 (e.g., whether the respiratory device 122 is on/off, the pressure of the air being delivered by the respiratory device 122, the temperature of the air being delivered by the respiratory device 122, etc.) and/or other information. For example, other information includes a sleep score and/or a therapy score (also referred to as a my Air^{™} score, such as described in WO 2016/061629), the current date/time, personal information for the user 210, etc. In some implementations, the display device 128 acts as a human-machine interface (HMI) that includes a graphic user interface (GUI) configured to display the image(s) as an input interface. The display device 128 can be an LED display, an OLED display, an LCD display, or the like. The input interface can be, for example, a touchscreen or touch-sensitive substrate, a mouse, a keyboard, or any sensor system configured to sense inputs made by a human user interacting with the respiratory device 122.

The humidification tank 129 is coupled to or integrated in the respiratory device 122. The humidification tank 129 includes a reservoir of water that can be used to humidify the pressurized air delivered from the respiratory device 122. The respiratory device 122 can include a heater to heat the water in the humidification tank 129 in order to humidify the pressurized air provided to the user. Additionally, in some implementations, the conduit 126 can also include a heating element (e.g., coupled to and/or imbedded in the conduit 126) that heats the pressurized air delivered to the user. The humidification tank 129 can be fluidly coupled to a water vapor inlet of the air pathway and deliver water vapor into the air pathway via the water vapor inlet, or can be formed in-line with the air pathway as part of the air pathway itself.

In some implementations, the system 100 can be used to deliver at least a portion of a substance from the receptacle 180 to the air pathway the user based at least in part on the physiological data, the sleep-related parameters, other data or information, or a combination thereof. Generally, modifying the delivery of the portion of the substance into the air pathway can include (i) initiating the delivery of the substance into the air pathway, (ii) ending the delivery of the portion of the substance into the air pathway, (iii) modifying an amount of the substance delivered into the air pathway, (iv) modifying a temporal characteristic of the delivery of the portion of the substance into the air pathway, (v) modifying a quantitative characteristic of the delivery of the portion of the substance into the air pathway, (vi) modifying any parameter associated with the delivery of the substance into the air pathway, or (vii) a combination of (i)-(vi).

Modifying the temporal characteristic of the delivery of the portion of the substance into the air pathway can include changing the rate at which the substance is delivered, starting and/or finishing at different times, continuing for different time periods, changing the time distribution or characteristics of the delivery, changing the amount distribution independently of the time distribution, etc. The independent time and amount variation ensures that, apart from varying the frequency of the release of the substance, one can vary the amount of substance released each time. In this manner, a number of different combination of release frequencies and release amounts (e.g., higher frequency but lower release amount, higher frequency and higher amount, lower frequency and higher amount, lower frequency and lower amount, etc.) can be achieved. Other modifications to the delivery of the portion of the substance into the air pathway can also be utilized.

The respiratory therapy system 120 can be used, for example, as a ventilator or a PAP system, such as, for example, (i) a continuous positive airway pressure (CPAP) system, (ii) an automatic positive airway pressure system (APAP), (iii) a bi-level or variable positive airway pressure system (BPAP or VPAP), or (iv) or a combination thereof. The CPAP system delivers a predetermined air pressure (e.g., determined by a sleep physician) to the user. The APAP system automatically varies the air pressure delivered to the user based on, for example, respiration data associated with the user. The BPAP or VPAP system is configured to deliver a first predetermined pressure (e.g., an inspiratory positive airway pressure or IPAP) and a second predetermined pressure (e.g., an expiratory positive airway pressure or EPAP) that is lower than the first predetermined pressure.

Still referring to FIG. 1, the one or more sensors 130 of the system 100 include a pressure sensor 132, a flow rate sensor 134, temperature sensor 136, a motion sensor 138, the microphone 140, the speaker 142, a radio-frequency (RF) receiver 146, a RF transmitter 148, a camera 150, an infrared sensor 152, a photoplethysmogram (PPG) sensor 154, an electrocardiogram (ECG) sensor 156, an electroencephalography (EEG) sensor 158, a capacitive sensor 160, a force sensor 162, a strain gauge sensor 164, an electromyography (EMG) sensor 166, an oxygen sensor 168, an analyte sensor 174, a moisture sensor 176, or a combination thereof. Generally, each of the one or more sensors 130 are configured to output sensor data that is received and stored in the memory device 114 or one or more other memory devices.

While the one or more sensors 130 are shown and described as including each of the pressure sensor 132, the flow rate sensor 134, the temperature sensor 136, the motion sensor 138, the microphone 140, the speaker 142, the RF receiver 146, the RF transmitter 148, the camera 150, the infrared sensor 152, the photoplethysmogram (PPG) sensor 154, the electrocardiogram (ECG) sensor 156, the electroencephalography (EEG) sensor 158, the capacitive sensor 160, the force sensor 162, the strain gauge sensor 164, the electromyography (EMG) sensor 166, the oxygen sensor 168, the analyte sensor 174, and the moisture sensor 176 more generally, the one or more sensors 130 can include a combination and any number of each of the sensors described and/or shown herein.

As described herein, the system 100 generally can be used to generate physiological data associated with a user (e.g., a user of the respiratory therapy system 120 shown in FIGS. 2A-2B) during a sleep session. The physiological data can be analyzed to generate one or more sleep-related parameters, which can include any parameter, measurement, etc. related to the user during the sleep session. The one or more sleep-related parameters that can be determined for the user 210 during the sleep session include, for example, an Apnea-Hypopnea Index (AHI) score, a sleep score, a flow signal, a respiration signal, a respiration rate, an inspiration amplitude, an expiration amplitude, an inspiration-expiration ratio, a number of events per hour, a pattern of events, a stage, pressure settings of the respiratory device 122, a heart rate, a heart rate variability, movement of the user 210, temperature, EEG activity, EMG activity, arousal, snoring, choking, coughing, whistling, wheezing, or a combination thereof.

In some implementations, the physiological data generated by one or more of the sensors 130 can be used by the control system 110 to determine a sleep-wake signal associated with the user 210 during the sleep session and one or more sleep-related parameters. The sleep-wake signal can be indicative of one or more sleep states, including wakefulness, relaxed wakefulness, micro-awakenings, and/or sleep stages, including a rapid eye movement (REM) stage, a first non-REM stage (often referred to as "N1"), a second non-REM stage (often referred to as "N2"), a third non-REM stage (often referred to as "N3"), or a combination thereof. Methods for determining sleep states and/or sleep stages from physiological data generated by one or more of the sensors, such as sensors 130, are described in, for example, WO 2014/047310, US 2014/0088373, WO 2017/132726, WO 2019/122413, and WO 2019/122414.

The sleep-wake signal can also be timestamped to determine a time that the user enters the bed, a time that the user exits the bed, a time that the user attempts to fall asleep, etc. The sleep-wake signal can be measured by the one or more sensors 130 during the sleep session at a predetermined sampling rate, such as, for example, one sample per second, one sample per 30 seconds, one sample per minute, etc. In some implementations, the sleep-wake signal can also be indicative of a respiration signal, a respiration rate, an inspiration amplitude, an expiration amplitude, an inspiration-expiration ratio, a number of events per hour, a pattern of events, pressure settings of the respiratory device 122, or a combination thereof during the sleep session. The event(s) can include snoring, apneas, central apneas, obstructive apneas, mixed apneas, hypopneas, a mask leak (e.g., from the user interface 124), a restless leg, a sleeping disorder, choking, an increased heart rate, labored breathing, an asthma attack, an epileptic event, a seizure, or a combination thereof. The one or more sleep-related parameters that can be determined for the user during the sleep session based on the sleep-wake signal include, for example, a total time in bed, a total sleep time, a sleep onset latency, a wake-after-sleep-onset parameter, a sleep efficiency, a fragmentation index, or a combination thereof.

Generally, the sleep session includes any point in time after the user 210 has laid or sat down in the bed 230 (or another area or object on which they intend to sleep), and has turned on the respiratory device 122 and donned the user interface 124. The sleep session can thus include time periods (i) when the user 210 is using the CPAP system but before the user 210 attempts to fall asleep (for example when the user 210 lays in the bed 230 reading a book); (ii) when the user 210 begins trying to fall asleep but is still awake; (iii) when the user 210 is in a light sleep (also referred to as stage 1 and stage 2 of non-rapid eye movement (NREM) sleep); (iv) when the user 210 is in a deep sleep (also referred to as slow-wave sleep, SWS, or stage 3 of NREM sleep); (v) when the user 210 is in rapid eye movement (REM) sleep; (vi) when the user 210 is periodically awake between light sleep, deep sleep, or REM sleep; or (vii) when the user 210 wakes up and does not fall back asleep.

The sleep session is generally defined as ending once the user 210 removes the user interface 124, turns off the respiratory device 122, and gets out of bed 230. In some implementations, the sleep session can include additional periods of time, or can be limited to only some of the above-disclosed time periods. For example, the sleep session can be defined to encompass a period of time beginning when the respiratory device 122 begins supplying the pressurized air to the airway or the user 210, ending when the respiratory device 122 stops supplying the pressurized air to the airway of the user 210, and including some or all of the time points in between, when the user 210 is asleep or awake.

The pressure sensor 132 outputs pressure data that can be stored in the memory device 114 and/or analyzed by the processor 112 of the control system 110. In some implementations, the pressure sensor 132 is an air pressure sensor (e.g., barometric pressure sensor) that generates sensor data indicative of the respiration (e.g., inhaling and/or exhaling) of the user of the respiratory therapy system 120 and/or ambient pressure. In such implementations, the pressure sensor 132 can be coupled to or integrated in the respiratory device 122. The pressure sensor 132 can be, for example, a capacitive sensor, an electromagnetic sensor, a piezoelectric sensor, a strain-gauge sensor, an optical sensor, a potentiometric sensor, or a combination thereof.

The flow rate sensor 134 outputs flow rate data that can be stored in the memory device 114 and/or analyzed by the processor 112 of the control system 110. In some implementations, the flow rate sensor 134 is used to determine an air flow rate from the respiratory device 122, an air flow rate through the conduit 126, an air flow rate through the user interface 124, or a combination thereof. In such implementations, the flow rate sensor 134 can be coupled to or integrated in the respiratory device 122, the user interface 124, or the conduit 126. The flow rate sensor 134 can be a mass flow rate sensor such as, for example, a rotary flow meter (e.g., Hall effect flow meters), a turbine flow meter, an orifice flow meter, an ultrasonic flow meter, a hot wire sensor, a vortex sensor, a membrane sensor, or a combination thereof.

The temperature sensor 136 outputs temperature data that can be stored in the memory device 114 and/or analyzed by the processor 112 of the control system 110. In some implementations, the temperature sensor 136 generates temperatures data indicative of a core body temperature of the user 210 (FIGS. 2A-2B), a skin temperature of the user 210, a temperature of the air flowing from the respiratory device 122 and/or through the conduit 126, a temperature in the user interface 124, an ambient temperature, or a combination thereof. The temperature sensor 136 can be, for example, a thermocouple sensor, a thermistor sensor, a silicon band gap temperature sensor or semiconductor-based sensor, a resistance temperature detector, or a combination thereof.

The motion sensor 138 outputs motion data that can be stored in the memory device 114 and/or analyzed by the processor 112 of the control system 110. The motion sensor 138 can be used to detect movement of the user 210 during the sleep session, and/or detect movement of any of the components of the respiratory therapy system 120, such as the respiratory device 122, the user interface 124, or the conduit 126. The motion sensor 138 can include one or more inertial sensors, such as accelerometers, gyroscopes, and magnetometers. In some implementations, the motion sensor 138 alternatively or additionally generates one or more signals representing bodily movement of the user, from which may be obtained a signal representing a sleep state of the user; for example, via a respiratory movement of the user. In some implementations, the motion data from the motion sensor 138 can be used in conjunction with additional data from another sensor 130 to determine the sleep state of the user.

The microphone 140 outputs sound data that can be stored in the memory device 114 and/or analyzed by the processor 112 of the control system 110. The microphone 140 can be used to record sound(s) during a sleep session (e.g., sounds from the user 210) to determine (e.g., using the control system 110) one or more sleep-related parameters, as described in further detail herein. The microphone 140 can be coupled to or integrated in the respiratory device 122, the user interface 124, the conduit 126, or the user device 170. In some implementations, the system 100 includes a plurality of microphones (e.g., two or more microphones and/or an array of microphones with beamforming) such that sound data generated by each of the plurality of microphones can be used to discriminate the sound data generated by another of the plurality of microphones.

The speaker 142 outputs sound waves that are audible to a user of the system 100 (e.g., the user 210 of FIGS. 2A-2B). The speaker 142 can be used, for example, as an alarm clock or to play an alert or message to the user 210 (e.g., in response to an event). The speaker 142 can be coupled to or integrated in the respiratory device 122, the user interface 124, the conduit 126, or the external device 170.

The microphone 140 and the speaker 142 can be used as separate devices. In some implementations, the microphone 140 and the speaker 142 can be combined into an acoustic sensor 141 (e.g., a SONAR sensor), as described in, for example, WO 2018/050913 and WO 2020/104465. In such implementations, the speaker 142 generates or emits sound waves at a predetermined interval and the microphone 140 detects the reflections of the emitted sound waves from the speaker 142. The sound waves generated or emitted by the speaker 142 have a frequency that is not audible to the human ear (e.g., below 20 Hz or above around 18 kHz) so as not to disturb the sleep of the user 210 or the bed partner 220 (FIGS. 2A-2B). Based at least in part on the data from the microphone 140 and/or the speaker 142, the control system 110 can determine a location of the user 210 (FIGS. 2A-2B) and/or one or more of the sleep-related parameters (e.g., a mouth leak status) described in herein such as, for example, a respiration signal, a respiration rate, an inspiration amplitude, an expiration amplitude, an inspiration-expiration ratio, a number of events per hour, a pattern of events, a sleep state, a sleep stage, pressure settings of the respiratory device 122, or any combination thereof. In this context, a sonar sensor may be understood to include an active acoustic sensing, such as by generating/transmitting ultrasound or low frequency ultrasound sensing signals (e.g., in a frequency range of about 17-23 kHz, 18-22 kHz, or 17-18 kHz, for example), through the air. Such a system may be considered in relation to WO2018/050913 and WO 2020/104465 mentioned above.

The RF transmitter 148 generates and/or emits radio waves having a predetermined frequency and/or a predetermined amplitude (e.g., within a high frequency band, within a low frequency band, long wave signals, short wave signals, etc.). The RF receiver 146 detects the reflections of the radio waves emitted from the RF transmitter 148, and this data can be analyzed by the control system 110 to determine a location of the user 210 (FIGS. 2A-2B) and/or one or more of the respiratory- and/or sleep-related parameters described herein. An RF receiver (either the RF receiver 146 and the RF transmitter 148 or another RF pair) can also be used for wireless communication between the control system 110, the respiratory device 122, the one or more sensors 130, the user device 170, or a combination thereof. While the RF receiver 146 and RF transmitter 148 are shown as being separate and distinct elements in FIG. 1, in some implementations, the RF receiver 146 and RF transmitter 148 are combined as a part of an RF sensor 147 (e.g., a RADAR sensor). In some such implementations, the RF sensor 147 includes a control circuit. The specific format of the RF communication could be Wi-Fi, Bluetooth, etc.

In some implementations, the RF sensor 147 is a part of a mesh system. One example of a mesh system is a Wi-Fi mesh system, which can include mesh nodes, mesh router(s), and mesh gateway(s), each of which can be mobile/movable or fixed. In such implementations, the Wi-Fi mesh system includes a Wi-Fi router and/or a Wi-Fi controller and one or more satellites (e.g., access points), each of which include an RF sensor that the is the same as, or similar to, the RF sensor 147. The Wi-Fi router and satellites continuously communicate with one another using Wi-Fi signals. The Wi-Fi mesh system can be used to generate motion data based on changes in the Wi-Fi signals (e.g., differences in received signal strength) between the router and the satellite(s) due to an object or person moving partially obstructing the signals. The motion data can be indicative of motion, breathing, heart rate, gait, falls, behavior, etc., or a combination thereof.

The camera 150 outputs image data reproducible as one or more images (e.g., still images, video images, thermal images, or a combination thereof) that can be stored in the memory device 114. The image data from the camera 150 can be used by the control system 110 to determine one or more of the sleep-related parameters described herein, such as, for example, one or more events (e.g., periodic limb movement or restless leg syndrome), a respiration signal, a respiration rate, an inspiration amplitude, an expiration amplitude, an inspiration-expiration ratio, a number of events per hour, a pattern of events, a sleep state, a sleep stage, or a combination thereof. Further, the image data from the camera 150 can be used to, for example, identify a location of the user, to determine chest movement of the user 210, to determine air flow of the mouth and/or nose of the user 210, to determine a time when the user 210 enters the bed 230, and to determine a time when the user 210 exits the bed 230.

The infrared (IR) sensor 152 outputs infrared image data reproducible as one or more infrared images (e.g., still images, video images, or both) that can be stored in the memory device 114. The infrared data from the IR sensor 152 can be used to determine one or more sleep-related parameters during a sleep session, including a temperature of the user 210 and/or movement of the user 210. The IR sensor 152 can also be used in conjunction with the camera 150 when measuring the presence, location, and/or movement of the user 210. The IR sensor 152 can detect infrared light having a wavelength between about 700 nm and about 1 mm, for example, while the camera 150 can detect visible light having a wavelength between about 380 nm and about 740 nm. In another example, the measurement is estimated by monitoring the acoustic reflection for a time period sufficient to capture time of flight to two or more sites of the physical obstruction, the physical obstruction having multiple sites extending through the airway of the user. In accordance with a specific example, the monitoring includes receiving and analyzing a reflection for a time period.

The PPG sensor 154 outputs physiological data associated with the user 210 (FIGS. 2A-2B) that can be used to determine one or more sleep-related parameters, such as, for example, a heart rate, a heart rate variability, a cardiac cycle, respiration rate, an inspiration amplitude, an expiration amplitude, an inspiration-expiration ratio, estimated blood pressure parameter(s), or a combination thereof. The PPG sensor 154 can be worn by the user 210, embedded in clothing and/or fabric that is worn by the user 210, embedded in and/or coupled to the user interface 124 and/or its associated headgear (e.g., straps, etc.), etc.

The ECG sensor 156 outputs physiological data associated with electrical activity of the heart of the user 210 (FIGS. 2A-2B). In some implementations, the ECG sensor 156 includes one or more electrodes that are positioned on or around a portion of the user 210 during the sleep session. The physiological data from the ECG sensor 156 can be used, for example, to determine one or more of the sleep-related parameters described herein.

The EEG sensor 158 outputs physiological data associated with electrical activity of the brain of the user 210. In some implementations, the EEG sensor 158 includes one or more electrodes that are positioned on or around the scalp of the user 210 during the sleep session. The physiological data from the EEG sensor 158 can be used, for example, to determine a sleep state of the user 210 at any given time during the sleep session. In some implementations, the EEG sensor 158 can be integrated in the user interface 124 and/or the associated headgear (e.g., straps, etc.).

The capacitive sensor 160, the force sensor 162, and the strain gauge sensor 164 output data that can be stored in the memory device 114 and used by the control system 110 to determine one or more of the sleep-related parameters described herein. The EMG sensor 166 outputs physiological data associated with electrical activity produced by one or more muscles. The oxygen sensor 168 outputs oxygen data indicative of an oxygen concentration of gas (e.g., in the conduit 126 or at the user interface 124). The oxygen sensor 168 can be, for example, an ultrasonic oxygen sensor, an electrical oxygen sensor, a chemical oxygen sensor, an optical oxygen sensor, or a combination thereof.

The analyte sensor 174 can be used to detect the presence of an analyte in the exhaled breath of the user 210. The data output by the analyte sensor 174 can be stored in the memory device 114 and used by the control system 110 to determine the identity and concentration of any analytes in the user 210's breath. In some implementations, the analyte sensor 174 is positioned near a mouth of the user 210 to detect analytes in breath exhaled from the user 210's mouth. For example, when the user interface 124 is a facial mask that covers the nose and mouth of the user 210, the analyte sensor 174 can be positioned within the facial mask to monitor the user 210's mouth breathing. In other implementations, such as when the user interface 124 is a nasal mask or a nasal pillow mask, the analyte sensor 174 can be positioned near the nose of the user 210 to detect analytes in breath exhaled through the user 210's nose. In still other implementations, the analyte sensor 174 can be positioned near the user 210's mouth when the user interface 124 is a nasal mask or a nasal pillow mask. In this implementation, the analyte sensor 174 can be used to detect whether any air is inadvertently leaking from the user 210's mouth. In some implementations, the analyte sensor 174 is a volatile organic compound (VOC) sensor that can be used to detect carbon-based chemicals or compounds. In some implementations, the analyte sensor 174 can also be used to detect whether the user 210 is breathing through their nose or mouth. For example, if the data output by an analyte sensor 174 positioned near the mouth of the user 210 or within the facial mask (in implementations where the user interface 124 is a facial mask) detects the presence of an analyte, the processor 112 can use this data as an indication that the user 210 is breathing through their mouth.

The moisture sensor 176 outputs data that can be stored in the memory device 114 and used by the control system 110. The moisture sensor 176 can be used to detect moisture in various areas surrounding the user (e.g., inside the conduit 126 or the user interface 124, near the user 210's face, near the connection between the conduit 126 and the user interface 124, near the connection between the conduit 126 and the respiratory device 122, etc.). Thus, in some implementations, the moisture sensor 176 can be positioned in the user interface 124 or in the conduit 126 to monitor the humidity of the pressurized air from the respiratory device 122. In other implementations, the moisture sensor 176 is placed near any area where moisture levels need to be monitored. The moisture sensor 176 can also be used to monitor the humidity of the ambient environment surrounding the user 210, for example the air inside the user 210's bedroom.

In some implementations, the one or more sensors 130 also include a galvanic skin response (GSR) sensor, a blood flow sensor, a respiration sensor, a pulse sensor, a sphygmomanometer sensor, an oximetry sensor, a sonar sensor, a RADAR sensor, a blood glucose sensor, a color sensor, a pH sensor, an air quality sensor, a tilt sensor, a LIDAR sensor, a rain sensor, a soil moisture sensor, a water flow sensor, an alcohol sensor, or a combination thereof.

While shown separately in FIG. 1, a combination of the one or more sensors 130 can be integrated in and/or coupled to any one or more of the components of the system 100, including the respiratory device 122, the user interface 124, the conduit 126, the humidification tank 129, the control system 110, the user device 170, or a combination thereof. For example, the acoustic sensor 141 and/or the RF sensor 147 can be integrated in and/or coupled to the user device 170. In such implementations, the user device 170 can be considered a secondary device that generates additional or secondary data for use by the system 100 (e.g., the control system 110) according to some aspects of the present disclosure. In some implementations, at least one of the one or more sensors 130 is not coupled to the respiratory device 122, the control system 110, or the user device 170, and is positioned generally adjacent to the user 210 during the sleep session (e.g., positioned on or in contact with a portion of the user 210, worn by the user 210, coupled to or positioned on the nightstand, coupled to the mattress, coupled to the ceiling, etc.).

The data from the one or more sensors 130 can be analyzed to determine one or more sleep-related parameters, which can include a respiration signal, a respiration rate, a respiration pattern, an inspiration amplitude, an expiration amplitude, an inspiration-expiration ratio, an occurrence of one or more events, a number of events per hour, a pattern of events, a sleep state, an apnea-hypopnea index (AHI), or any combination thereof. The one or more events can include snoring, apneas, central apneas, obstructive apneas, mixed apneas, hypopneas, a mask leak, a cough, a restless leg, a sleeping disorder, choking, an increased heart rate, labored breathing, an asthma attack, an epileptic event, a seizure, increased blood pressure, or any combination thereof. Many of these sleep-related parameters are physiological parameters, although some of the sleep-related parameters can be considered to be non-physiological parameters. Other types of physiological and non-physiological parameters can also be determined, either from the data from the one or more sensors 130, or from other types of data.

The user device 170 (FIG. 1) includes a display device 128. The user device 170 can be, for example, a mobile device such as a smart phone, a tablet, a gaming console, a smart watch, a laptop, or the like. Alternatively, the user device 170 can be an external sensing system, a television (e.g., a smart television) or another smart home device (e.g., a smart speaker(s) such as Google Home, Amazon Echo, Alexa etc.). In some implementations, the user device is a wearable device (e.g., a smart watch). The display device 172 is generally used to display image(s) including still images, video images, or both. In some implementations, the display device 172 acts as a human-machine interface (HMI) that includes a graphic user interface (GUI) configured to display the image(s) and an input interface. The display device 172 can be an LED display, an OLED display, an LCD display, or the like. The input interface can be, for example, a touchscreen or touch-sensitive substrate, a mouse, a keyboard, or any sensor system configured to sense inputs made by a human user interacting with the user device 170. In some implementations, one or more user devices can be used by and/or included in the system 100.

While the control system 110 and the memory device 114 are described and shown in FIG. 1 as being a separate and distinct component of the system 100, in some implementations, the control system 110 and/or the memory device 114 are integrated in the user device 170 and/or the respiratory device 122. Alternatively, in some implementations, the control system 110 or a portion thereof (e.g., the processor 112) can be located in a cloud (e.g., integrated in a server, integrated in an Internet of Things (IoT) device, connected to the cloud, be subject to edge cloud processing, etc.), located in one or more servers (e.g., remote servers, local servers, etc., or a combination thereof.

While system 100 is shown as including all of the components described above, more or fewer components can be included in a system for implementing the present disclosure. For example, a first alternative system includes the control system 110, the memory device 114, and at least one of the one or more sensors 130. As another example, a second alternative system includes the control system 110, the memory device 114, at least one of the one or more sensors 130, and the user device 170. As yet another example, a third alternative system includes the control system 110, the memory device 114, the respiratory therapy system 120, at least one of the one or more sensors 130, and the user device 170. Thus, various systems for implementing the present disclosure can be formed using any portion or portions of the components shown and described herein and/or in combination with one or more other components.

Generally, a user who is prescribed usage of a respiratory system will tend to experience higher quality sleep and less fatigue during the day after using the respiratory therapy system 120 during the sleep compared to not using the respiratory therapy system 120 (especially when the user suffers from sleep apnea or other sleep related disorders). However, many users do not conform to their prescribed usage because the user interface 124 is uncomfortable or cumbersome, or due to other side effects (e.g., dry mouth, dry lips, dry throat, discomfort, etc.). Users are more likely to fail to use the respiratory therapy system 120 as prescribed (or discontinue usage altogether) if they fail to perceive that they are experiencing any benefits (e.g., less fatigue during the day).

However, the side effects and/or the lack of improvement in sleep quality may be due to mouth leak rather than a lack of efficacy to the treatment. Thus, it is advantageous to characterize or determine a physical obstruction in the airway of the user and communicate information regarding the obstruction to the user or a third party to aid the user in obtaining higher quality sleep and/or improved treatment of sleep disordered breathing (SDB) involving apnea and/or hypopnea events, so that the user does not discontinue or reduce their usage of the respiratory therapy system 120 due to a perceived lack of benefit(s).

Referring generally to FIGS. 2A-2B, a portion of the system 100 (FIG. 1), according to some implementations, is illustrated. A user 210 of the respiratory therapy system 120 and a bed partner 220 are located in a bed 230 and are laying on a mattress 232. The user interface 124 (e.g., a full facial mask in FIG. 2A or a nasal mask in FIG. 2B) can be worn by the user 210 during a sleep session. The user interface 124 is fluidly coupled and/or connected to the respiratory device 122 via the conduit 126. In turn, the respiratory device 122 delivers pressurized air to the user 210 via the conduit 126 and the user interface 124 to increase the air pressure in the throat of the user 210 to aid in preventing the airway from closing and/or narrowing during sleep. The respiratory device 122 can be positioned on a nightstand 240 that is directly adjacent to the bed 230 as shown in FIG. 2A, or more generally, on any surface or structure that is generally adjacent to the bed 230 and/or the user 210.

In some implementations, the control system 110, the memory 114, any of the one or more sensors 130, or a combination thereof can be located on and/or in any surface and/or structure that is generally adjacent to the bed 230 and/or the user 210. For example, in some implementations, at least one of the one or more sensors 130 can be located at a first position 255A on and/or in one or more components of the respiratory therapy system 120 adjacent to the bed 230 and/or the user 210. The one or more sensors 130 can be coupled to the respiratory therapy system 120, the user interface 124, the conduit 126, the display device 128, the humidification tank 129, or a combination thereof.

Alternatively or additionally, at least one of the one or more sensors 130 can be located at a second position 255B on and/or in the bed 230 (e.g., the one or more sensors 130 are coupled to and/or integrated in the bed 230). Further, alternatively or additionally, at least one of the one or more sensors 130 can be located at a third position 255C on and/or in the mattress 232 that is adjacent to the bed 230 and/or the user 210 (e.g., the one or more sensors 130 are coupled to and/or integrated in the mattress 232). Alternatively or additionally, at least one of the one or more sensors 130 can be located at a fourth position 255D on and/or in a pillow that is generally adjacent to the bed 230 and/or the user 210.

Alternatively or additionally, at least one of the one or more sensors 130 can be located at a fifth position 255E on and/or in the nightstand 240 that is generally adjacent to the bed 230 and/or the user 210. Alternatively or additionally, at least one of the one or more sensors 130 can be located at a sixth position 255F such that the at least one of the one or more sensors 130 are coupled to and/or positioned on the user 215 (e.g., the one or more sensors 130 are embedded in or coupled to fabric, clothing 212, and/or a smart device 270 worn by the user 210). More generally, at least one of the one or more sensors 130 can be positioned at any suitable location relative to the user 210 such that the one or more sensors 130 can generate sensor data associated with the user 210.

In some implementations, a primary sensor, such as the microphone 140, is configured to generate acoustic data associated with the user 210 during a sleep session. For example, one or more microphones (the same as, or similar to, the microphone 140 of FIG. 1) can be integrated in and/or coupled to (i) a circuit board of the respiratory device 122, (ii) the conduit 126, (iii) a connector between components of the respiratory therapy system 120, (iv) the user interface 124, (v) a headgear (e.g., straps) associated with the user interface, or (vi) a combination thereof.

Additionally or alternatively, one or more microphones (the same as, or similar to, the microphone 140 of FIG. 1) can be integrated in and/or coupled to a co-located smart device, such as the user device 170, a TV, a watch (e.g., a mechanical watch or the smart device 270), a pendant, the mattress 232, the bed 230, beddings positioned on the bed 230, the pillow, a speaker (e.g., the speaker 142 of FIG. 1), a radio, a tablet, a waterless humidifier, or a combination thereof.

Additionally or alternatively, in some implementations, one or more microphones (the same as, or similar to, the microphone 140 of FIG. 1) can be remote from the system 100 (FIG. 1) and/or the user 210 (FIGS. 2A-2B), so long as there is an air passage allowing acoustic signals to travel to the one or more microphones. For example, the one or more microphones can be in a different room from the room containing the system 100. Based at least in part on an analysis of the acoustic data, a physical obstruction in the airway of a user can be characterize and/or determined.

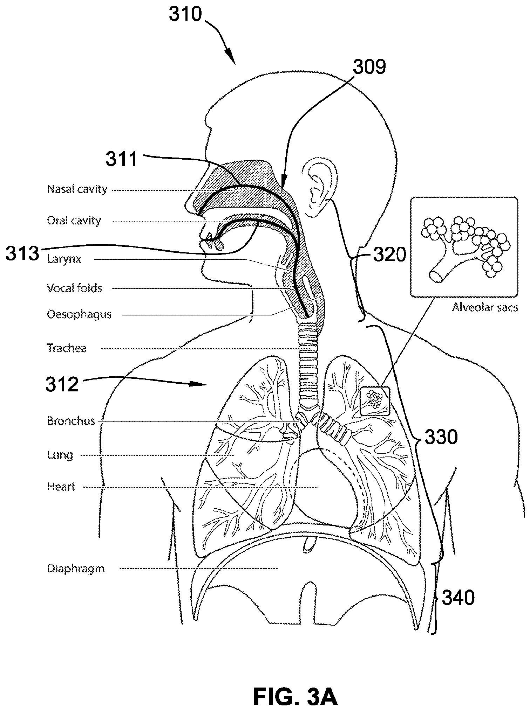

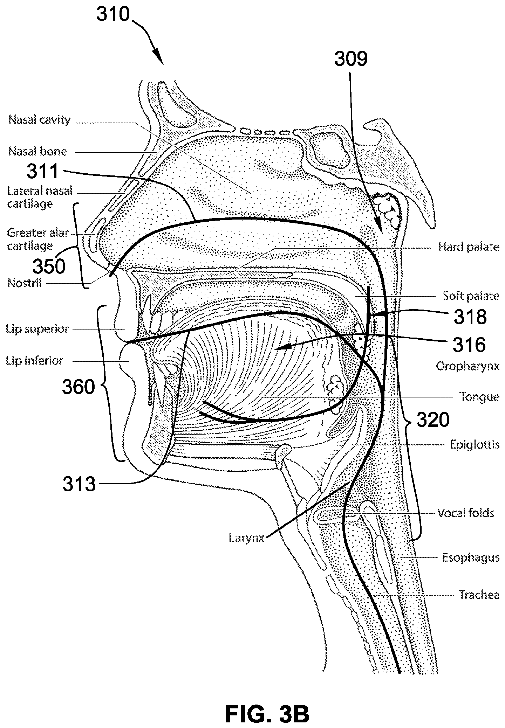

Referring to FIGs. 3A and 3B, an overview shows an airway 309 of a respiratory system 312 of a user 310 (e.g., a patient). The airway 309 includes, more specifically, a nasal airway 311 and a mouth airway 313. The user 310 generally includes a nasal cavity, an oral cavity, a larynx, vocal folds, an esophagus, a trachea, a bronchus, lungs, alveolar sacs, a heart, and a diaphragm. More generally, the user 310 has a throat 320, which includes a region(s) of the respiratory system 312 of the user 310 generally in the neck area of the user 310. The diaphragm of the user 310 is a sheet of muscle that extends across the bottom of the rib cage of the user 310. The diaphragm generally separates the thoracic cavity 330 of the user 310, which contains the heart, lungs, and ribs, from the abdominal cavity 340 of the user 310. As the diaphragm contracts, the volume of the thoracic cavity 330 increases and air is drawn into the lungs. A hypoglossal nerve 318 is generally involved in controlling movement of the tongue 316 and includes a plurality of nerve branches distributed to the extrinsic and intrinsic muscles of the tongue 316.

Referring more specifically to FIG. 3B, the airway 309 of the user 310 includes the nasal cavity, nasal bone, lateral nasal cartilage, greater alar cartilage, nostrils (one shown), a lip superior, a lip inferior, the larynx, a hard palate, a soft palate, an oropharynx, a tongue, an epiglottis, the vocal folds, the esophagus, and the trachea. The respiratory system 312 of the user 310 facilitates gas exchange. The nose 350 and mouth 360 of the user 310 form the entrance, respectively, to the airway 309 of the user 310. As best shown in FIG. 3A, the airway 309 include a series of branching tubes, which become narrower, shorter, and more numerous as they penetrate deeper into the lungs of the user 310. The prime function of the lungs is gas exchange, allowing oxygen to move from the inhaled air into the venous blood and carbon dioxide to move in the opposite direction. The trachea divides into right and left main bronchi, which further divide eventually into terminal bronchioles. The bronchi make up conducting airways, and do not take part in gas exchange. Further divisions of the airway 309 leads to the respiratory bronchioles, and eventually to the alveoli. The alveolated region of the lungs is where the gas exchange takes place, and is referred to as the respiratory zone.

A range of respiratory disorders exist that can impact the user 310. Certain disorders are characterized by particular events (e.g., apneas, hypopneas, hyperpneas, or any combination thereof). Examples of sleep-related and/or respiratory disorders include Periodic Limb Movement Disorder (PLMD), Restless Leg Syndrome (RLS), Sleep-Disordered Breathing (SDB), Obstructive Sleep Apnea (OSA), Cheyne-Stokes Respiration (CSR), respiratory insufficiency, Obesity Hyperventilation Syndrome (OHS), Chronic Obstructive Pulmonary Disease (COPD), Neuromuscular Disease (NMD), and chest wall disorders.

Obstructive Sleep Apnea (OSA), which is a form of Sleep Disordered Breathing (SDB), is characterized by events including occlusion or obstruction of the upper air passage during sleep resulting from a combination of an abnormally small upper airway 309 and the normal loss of muscle tone in the region of the tongue, soft palate and posterior oropharyngeal wall. The condition causes the affected patient to stop breathing for periods typically of 30 to 120 seconds in duration, sometimes 200 to 300 times per night. OSA often causes excessive daytime somnolence, and it may cause cardiovascular disease and brain damage. The syndrome is a common disorder, particularly in middle aged overweight males, although a person affected may have no awareness of the problem.

Cheyne-Stokes Respiration (CSR) is another form of sleep disordered breathing. CSR is a disorder of a patient's respiratory controller in which there are rhythmic alternating periods of waxing and waning ventilation known as CSR cycles. CSR is characterized by repetitive de-oxygenation and re-oxygenation of the arterial blood. It is possible that CSR is harmful because of the repetitive hypoxia. In some users, CSR is associated with repetitive arousal from sleep, which causes severe sleep disruption, increased sympathetic activity, and increased afterload.

Respiratory failure is an umbrella term for respiratory disorders in which the lungs are unable to inspire sufficient oxygen or exhale sufficient CO₂ to meet the user's needs. Respiratory failure may encompass some or all of the following disorders. A user with respiratory insufficiency (a form of respiratory failure) may experience abnormal shortness of breath on exercise.

Obesity Hyperventilation Syndrome (OHS) is defined as the combination of severe obesity and awake chronic hypercapnia, in the absence of other known causes for hypoventilation. Symptoms include dyspnea, morning headache and excessive daytime sleepiness.

Chronic Obstructive Pulmonary Disease (COPD) encompasses any of a group of lower airway diseases that have certain characteristics in common, such as increased resistance to air movement, extended expiratory phase of respiration, and loss of the normal elasticity of the lung. Examples of COPD are emphysema and chronic bronchitis. COPD is caused by chronic tobacco smoking (primary risk factor), occupational exposures, air pollution and genetic factors. Symptoms include: dyspnea on exertion, chronic cough and sputum production.

Neuromuscular Disease (NMD) encompasses many diseases and ailments that impair the functioning of the muscles either directly via intrinsic muscle pathology, or indirectly via nerve pathology. Some users suffering from NMD are characterized by progressive muscular impairment leading to loss of ambulation, being wheelchair-bound, swallowing difficulties, respiratory muscle weakness and, eventually, death from respiratory failure. Neuromuscular disorders can be divided into rapidly progressive and slowly progressive: (i) rapidly progressive disorders: characterized by muscle impairment that worsens over months and results in death within a few years (e.g. amyotrophic lateral sclerosis (ALS) and Duchenne muscular dystrophy (DMD) in teenagers); (ii) variable or slowly progressive disorders: characterized by muscle impairment that worsens over years and only mildly reduces life expectancy (e.g. limb girdle, Facioscapulohumeral and myotonic muscular dystrophy). Symptoms of respiratory failure in NMD include: increasing generalized weakness, dysphagia, dyspnea on exertion and at rest, fatigue, sleepiness, morning headache, and difficulties with concentration and mood changes.

Chest wall disorders are a group of thoracic deformities that result in inefficient coupling between the respiratory muscles and the thoracic cage. The disorders are usually characterized by a restrictive defect and share the potential of long term hypercapnic respiratory failure. Scoliosis and/or kyphoscoliosis may cause severe respiratory failure. Symptoms of respiratory failure include: dyspnea on exertion, peripheral edema, orthopnea, repeated chest infections, morning headaches, fatigue, poor sleep quality and loss of appetite.

These other disorders are characterized by particular events (e.g., snoring, an apnea, a hypopnea, a restless leg, a sleeping disorder, choking, an increased heart rate, labored breathing, an asthma attack, an epileptic event, a seizure, or any combination thereof) that occur when the individual is sleeping. While these other sleep-related disorders may have similar symptoms as insomnia, distinguishing these other sleep-related disorders from insomnia is useful for tailoring an effective treatment plan distinguishing characteristics that may call for different treatments. For example, fatigue is generally a feature of insomnia, whereas excessive daytime sleepiness is a characteristic feature of other disorders (e.g., PLMD) and reflects a physiological propensity to fall asleep unintentionally.

One or more of the disorders and associated symptoms discussed above can be better treated if the cause is related to a physical obstruction within the airway 309. Determining characteristics, such as the location and size, of the physical obstruction are extremely beneficial in recommending and/or improving treatment of the affected person.

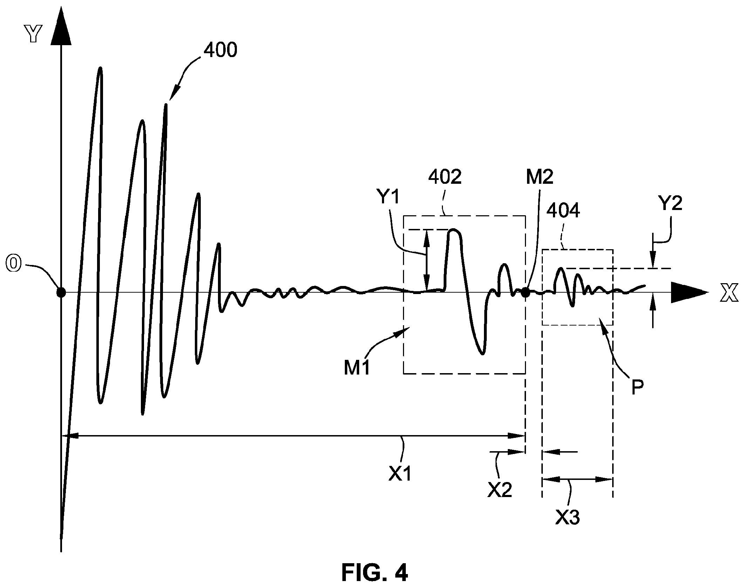

Referring to FIG. 4, a cepstrum illustrates an acoustic signal 400 with a varying acoustic amplitude along a Y-axis as the acoustic signal travel along an X-axis. The acoustic signal 400 has an acoustic amplitude that varies based generally on any encountered physical obstructions. The cepstrum is obtained via one or more signal processing methods.

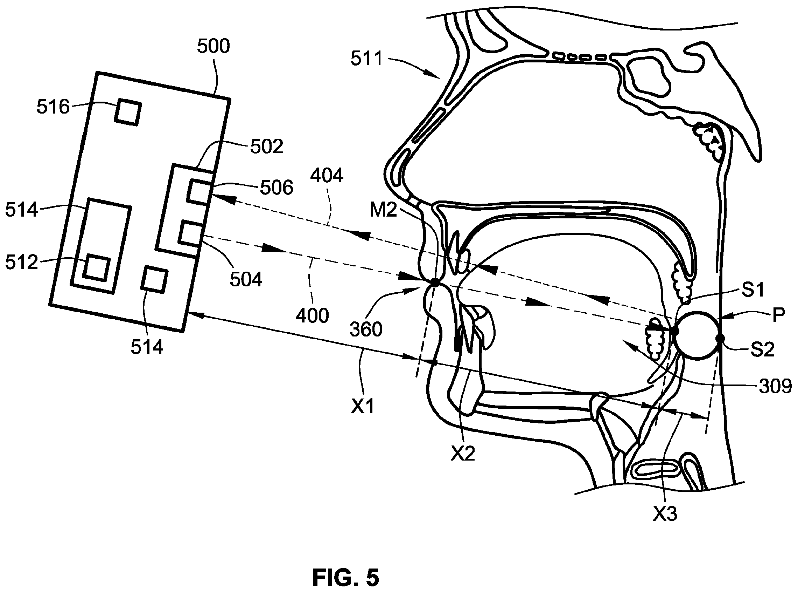

The traveled path along the X-axis includes a pre-airway portion X1 that refers to a distance of the acoustic signal 400 up to and including a mask location M1 of a user 511 (illustrated in FIG 5). At the mask location M1, the acoustic signal 400 has a first amplitude Y1 that is indicative at least in part of a first acoustic reflection 402, which is caused by a mask at the mask location M1. The distance between a point of origin O of sound emission and the mask location M1 is indicated as a first distance X1. The mask location M1 ends at a mouth location M2.

Then, after encountering the mouth location M2, the acoustic signal 400 further travels into the airway 309 of the user a second distance X2 and encounters a physical obstruction P. At the physical obstruction P, the acoustic signal has a second amplitude Y2 that is indicative at least in part of a second acoustic reflection 404 (also referred to as "the acoustic reflection 404"), which is caused by the physical obstruction P. The physical obstruction P extends into the airway 309 a third distance X3 after the second distance X2. Thus, the cepstrum identifies a distance (e.g., the first distance X1, the second distance X2, and or the third distance X3) that is associated with the first and second acoustic reflections 402, 404, the distance showing the location of the physical obstruction P within the airway 309 of the user 511 (illustrated in FIG. 5).

The mask, according to some examples, is in the form of the user interface 124 described above and illustrated in FIG. 2A. For example, the mask is a facial mask that is positioned to cover the nose and mouth of the user. When the mask is placed at the mouth location M2, the cepstrum identifies the second distance X2, which is the distance between the mask and the physical obstruction P.

The first acoustic reflection 402 has a specific signal morphology that is different than the signal morphology of the second acoustic reflection 404. For example, the first amplitude Y1 is greater than the second amplitude Y2. In another example, the shape of the first acoustic reflection 402 is different than the shape of the second acoustic reflection 404. According to some examples, the signal morphology includes an acoustic shape of an acoustic wave.

According to some implementations of the present disclosure, one or more of the physical characteristics of the physical obstruction P are estimated based on the signal morphology of one or more of the first acoustic reflection 402 and the second acoustic reflection 404.

Referring to FIG. 5, a system 500 includes an acoustic device 502 for generating the acoustic signal 400. The acoustic device 502 includes a sound generator 504 and a sound detector 506. In some implementations, the sound detector 506 may act as a passive acoustic sensor suitable for suitable for generating passive acoustic data associated with the breathing of a user.

According to some implementations of the present disclosure, the sound generator 504 is in the form of a speaker or a motor. For example, the speaker is the speaker 142 described above and illustrated in FIG. 1. In another example, the motor is included in the respiratory device 122 described above and illustrated in FIGs. 1 and 2A. Thus, for example the acoustic signal is emitted by either or both of a speaker or motor 502.

According to some implementations of the present disclosure, at least one of the sound generator 504 and the sound detector 506 is not necessarily included in the acoustic device 502, but is generally included anywhere in the system 500. According to some implementations, the system 500 is generally similar or identical to the system 100 that is described above and is illustrated in FIG. 1, and includes one or more components of the system 100.

According to some implementations of the present disclosure, the speaker 502 generates the acoustic signal 400 in the form of a sound, the sound being one or more of a standard sound (e.g., an original, unmodified sound from an off-the-shelf sound application), a custom sound, an inaudible frequency, a white noise sound, a broad band impulse, a continuous sinusoidal waveform, a square waveform, a sawtooth waveform, and a frequency modulated sinusoid (e.g., chirp). According to some other implementations, the acoustic signal 400 is in the form of one or more of an audible sound, an ultrasonic sound, a white noise signal, a Gaussian white noise signal, a brown noise signal, a pink noise signal, a wide-band signal, a narrow-band signal, or combinations thereof.

According to some implementations of the present disclosure, the sound detector 506 is in the form of a microphone. For example, the microphone is the microphone 140 described above and illustrated in FIG. 1.

The system 500 can further include a memory device 514 for storing machine-readable instructions. According to some implementations, the memory device 514 is in the form of the memory device 114 described above and illustrated in FIG. 1.

The system 500 can further include a control system 510 having one or more processors 512. The processors 512 are communicatively coupled to the acoustic device 502. According to some implementations of the present disclosure, the control system 510 is in the form of the control system 110 described above and illustrated in FIG. 1. According to some implementations of the present disclosure, the processors 512 are in the form of the processors 112 described above and illustrate in FIG. 1.

The control system 510 is configured to execute the machine-readable instructions stored in the memory device 514 to detect the acoustic reflection 404 of the acoustic signal 400 emitted by the acoustic device 502 into the airway 309 of a user 511. The acoustic reflection 404 is caused by the physical obstruction P within the airway 309 of the user 511.

According to some implementations, the system 500 optionally includes a motion sensor 516 for detecting a position of the user 511. According to some implementations of the present disclosure, the motion sensor 516 is in the form of the motion sensor 138 described above and illustrated in FIG. 1. The motion sensor 516 includes, optionally, one or more of a camera, a bed sensor, an accelerometer, a sonar sensor, a radio frequency (RF) sensor such as an impulse-radio ultra wideband (IR-UWB) sensor, and/or any other sensors described above in reference to the motion sensor 138, acoustic sensor 141 or RF sensor 147.

The motion sensor 516 detects a user position, including a user location and/or a user orientation. For example, the user location identifies the user position relative to the system 500 and/or relative to a bed (such as the bed 230 described above and illustrated in FIG. 2A). In another example, the user location identifies the user orientation relative to the system 500 and/or relative to the bed.

Acoustic data associated with the acoustic reflection 404 is analyzed, and an occurrence of the physical obstruction P in the airway 309 of the user 511 is characterized. The characterization of the occurrence of the physical obstruction P is based at least in part on the analyzed acoustic data. According to some implementations of the present disclosure, the characterization is based solely on the analyzed acoustic data. That is, in some implementations, the methods and systems disclosed herein are capable of characterizing an apnea event based solely on the analyzed acoustic data. The physical obstruction P is indicative of an apnea event or a hypopnea event in the user 511. Analysis of acoustic reflections to detect obstructions in a conduit of a respiratory system is disclosed in WO 2010/091462. Similar analysis may be employed in the present case to characterize a physical obstruction in the airway of an individual user, wherein the physical obstruction is an obstructive apnea, a central apnea or a hypopnea.

According to some implementations of the present disclosure, the acoustic data includes a physical measurement of at least one of a mouth area, a nose area, and a throat area of the user 511 (which are more clearly illustrated in FIGs. 3A and 3B).

The characterization of the occurrence of the physical obstruction P includes, for example, determining a site location of the physical obstruction P within the airway 309 of the user 511. According to the illustration of FIG. 5, the physical obstruction P is after the second distance X2 from the point of entry of the acoustic signal into the user's airway 309, e.g., the mouth 360 of the user 511. The characterization further includes, according to other examples, determining the site size, site shape, and/or any other physical characteristics of the physical obstruction P.

According to some implementations of the present disclosure, one or more of the physical characteristics of the physical obstruction P are estimated based on a change in acoustic impedance, as indicated by the analyzed acoustic data. According to some implementations of the present disclosure, one or more of the physical characteristics of the physical obstruction P are estimated based on analyzing the acoustic reflection 404 that occurs from the physical obstruction P within the airway 309 of the user 511.

According to some implementations of the present disclosure, one or more of the physical characteristics of the physical obstruction P are estimated based on an analysis of (a) one or more acoustic waves reflecting at the mouth location M2 (before an entry into the airway 309 of the user 511), and (b) one or more acoustic waves reflecting at the physical obstruction P (after the entry into the airway 309 of the user 511). According to some other implementations of the present disclosure, one or more of the physical characteristics of the physical obstruction P are estimated based on the second distance X2 that acoustic waves travel into the airway 309 of the user 511 past the mouth location M2. According to some examples, the entry point into the airway 309 is a mouth area (i.e., at the mouth location M2) or a nose area of the user 511.

According to some implementations of the present disclosure, one or more of the physical characteristics of the physical obstruction P are estimated based on one or more characteristics of the user 511, such as age, sex, weight, mouth size, neck size, or type of mask currently used by the user 511.

According to some implementations of the present disclosure, the acoustic signal 400 is emitted at specific monitoring times after detecting that an apnea event or a hypopnea event is occurring, e.g., after detecting using a respiratory device that an apnea event or a hypopnea event is occurring. For example, the specific monitoring times are selected to be at intervals of 0.1 seconds for a duration of at least 4 seconds.

According to some implementations of the present disclosure, the acoustic signal 400 is emitted intermittently. For example, the acoustic signal 400 is emitted every 0.01 seconds to every 10 seconds of a predetermined time period. According to one example, the predetermined time period is between 10:00 pm and 6:00 am, during a typical sleeping period of the user 511.

According to some implementations of the present disclosure, the acoustic signal 400 is emitted continuously. For example, the acoustic signal 400 is emitted continuously during a predetermined time period. According to one example, the predetermined time period is between 10:00 pm and 6:00 am, during a typical sleeping period of the user 511.

According to some implementations of the present disclosure, the acoustic reflection 404 is monitored for a time period sufficient to capture time of flight to two or more sites of the physical obstruction P. For example, the physical obstruction P has multiple sites that include a first site S1 and a second site S2 extending through the airway 309 of the user 511. In other words, the physical obstruction P is not necessarily restricted to a single point in the airway 309, but optionally extends along the airway 309.

According to some implementations of the present disclosure, a minimum time period for monitoring the acoustic reflection 404 is at least about 0.015 seconds from the time when the acoustic signal 400 is emitted. According to other implementations of the present disclosure, the minimum time period is at least about 4 seconds from the time when the acoustic signal 400 is emitted.

According to some implementations of the present disclosure, the acoustic data is sampled during an entire apnea event or hypopnea event. For example, a typical apnea event is defined as cessation of breathing for a minimum of 10 seconds. However, the apnea event may last between about 10 seconds and about 30 seconds. In some cases, the apnea event may be over a minute. Accordingly, the acoustic data, assuming that monitoring of the physical obstruction P starts at the onset of the apnea event, is sampled for a minimum of 10 seconds, until the apnea event ends. According to an exemplary implementation of the present disclosure, a duration of the physical obstruction P is determined by monitoring and determining the onset and the end of the physical obstruction P.

According to some implementations of the present disclosure, the occurrence of the physical obstruction P is characterized within about 0.25 seconds from the onset of the physical obstruction. Thus, according to this example, the characterization of the physical obstruction P (e.g., location and size) is achieved very quickly.

According to some implementations of the present disclosure, the occurrence of the physical obstruction P is characterized after approximately 4-6 seconds from the start of the apnea event or the hypopnea event. The respective event is optionally determined independently with a different system, such as the respiratory therapy system 120 described above in reference to FIGs. 1, 2A, and 2B. For example, the respiratory therapy system 120 is a PAP system, a CPAP system, a BPAP system, a VPAP system, or a combination thereof.

According to some implementations of the present disclosure, the system 500 includes the respiratory therapy system 120 (illustrated in FIGs. 1, 2A, and 2B) in the form of the PAP system 120 for treating obstructive sleep apnea. Optionally, the PAP system 120 is capable of independently determining an occurrence of an apnea event or hypopnea event, as discussed above in reference to FIGs. 1, 2A, and 2B.

According to some implementations, as discussed above and illustrates in FIGs. 1, 2A, and 2B, the PAP system 120 includes a respiratory device 122 that delivers pressurized air to a user. Flow data and/or pressure data associated with the pressurized air is used to independently determine the occurrence of the apnea event or hypopnea event. The PAP system 120 includes at least one of an external pressure sensor and/or an internal pressure sensor for outputting the flow data and/or the pressure data. The external pressure sensor and/or the internal pressure are optionally in the form of the pressure sensor 132 and/or the flow rate sensor 134 (described above and illustrated in FIG. 1).

The PAP system 120, as incorporated in the system 500, optionally includes a user interface similar to or identical to the user interface 124 described above and illustrates in FIGs. 1, 2A, and 2B for positioning on the user 511. The user interface includes, a nasal mask, a nasal pillow mask, and/or a full-face mask.

According to some implementations in which the system 500 includes the respiratory therapy system 120 (illustrated in FIGs. 1, 2A, and 2B), the control system 500 is configured to supply pressurized air via the respiratory therapy system 120 to the airway 309 of the user 511 during a sleep session. The control system 500 receives flow data associated with the pressurized air (e.g., via the flow rate sensor 134 illustrated in FIG. 1), and determines, based on the flow data, an occurrence of an apnea event or a hypopnea event. The control system 500 emits the acoustic signal 400, via the acoustic device 502, into the airway 309 of the user 511 during the apnea event or the hypopnea event. The acoustic reflection 404 is received based on the physical obstruction P within the airway 309 of the user 511. Acoustic data associated with the acoustic reflection 404 is analyzed, and physical characteristics of the physical obstruction P are characterized based, at least in part, on the analyzed acoustic data.

According to some implementations of the present disclosure, the acoustic reflection may be processed to decouple reflections in space and/or time by estimating a cepstrum signal. This can be achieved by processing the acoustic reflection in a series of stages: calculate the frequency spectrum of the acoustic reflection via a fast Fourier transform; take the natural logarithm of the absolute magnitude of this frequency spectrum; and perform the inverse Fourier transform and calculate the real part of the signal to produce a cepstrum. This process may be repeated over multiple time intervals and an average cepstrum may be estimated. The resulting cepstrum signal transforms the acoustic response into the quefrency domain which is analogous to separating reflections in time and/or distance. The cepstrum signal could then be further processed to characterize the physical obstruction using one or more statistical analysis, pattern recognition algorithms, and/or machine learning or deep learning techniques.

According to some implementations of the present disclosure, the acoustic data includes a plurality of audio samples. According to some further implementations of the present disclosure, the multiple audio samples are averaged to provide an average result of the acoustic reflection 404. The audio samples correspond to a plurality of acoustic reflections received over a period of time, such as a predetermined period of time.

According to some implementations of the present disclosure, the control system 510 is further configured to identify a location of a body organ of the user 511, e.g., a lung. Thus, instead of or in addition to identifying the physical obstruction P, the control system 510 provides an additional medical benefit of identifying other body organs. By identifying a lung(s) and the location thereof based on the reflected acoustic signal, one can better estimate the location of a physical obstruction in the airway.

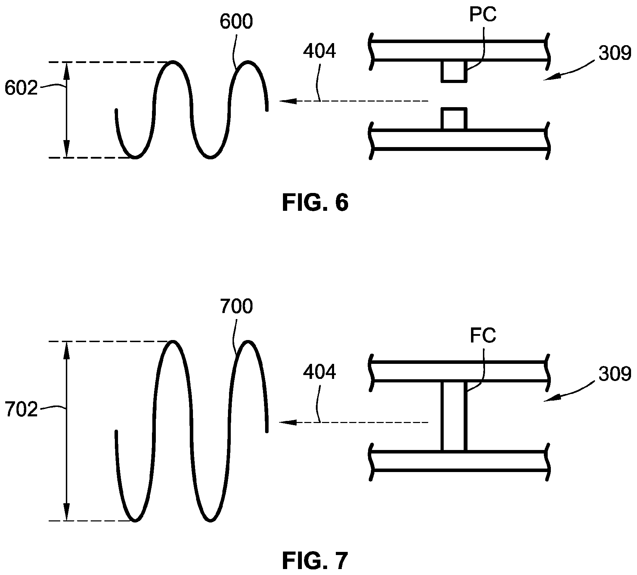

Referring to FIG. 6, according to some implementations of the present disclosure, a determination is made that the airway 309 is in a partially collapsed state PC. The partially collapsed state PC indicates that a hypopnea event is occurring. Acoustic data shows that the acoustic reflection 404 is represented having a first shape 600 with a first amplitude 602.

Referring to FIG. 7, according to some implementations of the present disclosure, a determination is made that the airway 309 is in a fully collapsed state FC. The fully collapsed state FC indicates that a hypopnea event is occurring. Acoustic data shows that the acoustic reflection 404 is represented having a second shape 700 with a second amplitude 702, which are different than the first shape 600 and the first amplitude 602 of the acoustic data associated with the hypopnea event described above and illustrated in FIG. 6.

The first amplitude 602 or the second amplitude 702 generally refer to features in a cepstrum signal, such as peaks or troughs. For purposes of analysis, any segment of a signal that deviates above or below zero and its amplitude are of relevance. For example, segment features of relevance include a segment, height, a segment width for any given peak, an area under a curve (and whether it is +ve or -ve). In reference to a physical obstruction, a +ve bump in the cepstrum signal is likely to take on a different shape that is indicative of the physical obstruction.

For example, occlusion of a conduit and signatures in the acoustic data includes baseline acoustic signatures that are determined from acoustic data for a headgear conduit under normal non-occluded operation during a therapy session, along with varying degrees of occlusion, and varying locations of occlusion within regions prone for occlusion for a headgear conduit. The baseline acoustic signatures can then be compared to actual acoustic signatures (e.g., cepstrums) to identify anomalies in a headgear conduit and determine that the anomaly is due to an occlusion. In some implementations, an estimate of the degree of occlusion in the headgear conduit can be estimated, such as assigning a percentage of blockage, or partial blockage, or full blockage. Baseline data for comparison to operational acoustic signatures can be stored in a digital library of baseline acoustic signatures and/or developed through training algorithms for acoustic data for a particular respiratory therapy system or from a collection of respiratory therapy systems.

In some implementations, it is contemplated that a determined signature can be analyzed by a machine learning algorithm having learned patterns associated with occluded and/or non-occluded headgear conduits during operation of a therapy session, along with learned patterns with varying degrees of occlusion, and varying locations of occlusion within regions prone for occlusion in a headgear conduit.

In some implementations, a method of detecting an occlusion is based on determining if an identified anomaly in a determined acoustic signature is an occlusion by inputting analyzed acoustic data into a machine learning model. In one or more implementations, the machine learning model can be supervised or unsupervised. In one or more implementations, the machine learning model can be a neural network. In one or more implementations, the neural network can be a deep neural network or a shallow neural network. In one or more implementations, the deep neural network can be a convolutional neural network. According to some aspects of the method, the deep neural network includes one or more convolutional layers and one or more max pooling layers.

In some implementations, one or more acoustic signatures can be fed into, for example, a machine learning model (e.g., linear regression, logistic regression, nearest neighbor, decision trees, PCA, naive Bayes classifier, and k-means clustering, random forest, etc.) and return an identification of an anomaly in a headgear conduit (e.g., occluded, non-occluded, degree of occlusion). In one or more implementations, determining if an identified anomaly in a determined acoustic signature relates to an occlusion of a headgear conduit can be based, at least in part, on one or more determined acoustic signatures matching one or more known signatures of headgear conduits of a headgear interface with a known degree of occlusion (e.g., fully occluded, partially occluded, non-occluded). The determination that an identified anomaly relates to an occlusion can be determined based on a matching determined signatures with known signatures.

In some implementations, it is contemplated that various features or signatures derived from generated acoustic data for an acoustic signal can be determined, such as a peak height or peak heights for a signal over time. Changes between a peak height for a non-occluded headgear conduit and a peak height for an occluded headgear conduit (or varying degrees of occlusion) can be analyzed. The analysis can then identify anomalies in a headgear conduit and determine that the anomaly is due to an occlusion. For example, if a peak height is above a certain threshold value, the analysis would determine that the anomaly is an occlusion in the headgear conduit. If the peak height is below a certain value, the analysis would determine the anomaly is not an occlusion in the headgear conduit.

The present technology contemplates that in response to determining an identified anomaly from a determined acoustic signature relates to an occlusion, information related to a detected occlusion in a headgear conduit is stored for analysis by a body position algorithm module and/or a body movement algorithm module associated with the respiratory therapy system. In some implementations, the information related to a detected occlusion in one or more headgear conduits can be inputted into a body position algorithm module and/or a body movement algorithm module associated with the respiratory therapy system. An occlusion of a headgear conduit would be expected to indicate that a user's head/body is in a side position/orientation.

In some implementations, an acoustic signature in the context of a cepstrum is contemplated to include a certain pattern appearing in the cepstrum. In determining is an anomaly (e.g., an occlusion) exists in a headgear conduit, the analysis can include looking at one cepstrum or many cepstrums (e.g., combined). A cepstrum can include multiple features that together can be indicative of a blocked headgear conduit. Cepstrum features of interest can include the location, the amplitude, and the shape of various bumps, peaks, and troughs in the cepstrum plot. For a conduit occlusion, a change in the cepstrum in a region on interest of the headgear conduit (e.g., regions 450a, 450b from FIG. 4) is typically characterized by a positive peak in the cepstrum plot corresponding to the point of occlusion (i.e. narrowing) of the headgear conduit. However, the conduit occlusion may additionally or alternatively be characterized by one or more negative peaks in the cepstrum plot corresponding to the points of widening of the acoustic pathway in the vicinity of the point of occlusion of the headgear conduit. Other features of interest can include the peak height (amplitude), the peak prominence, the skewness, the kurtosis, areas above/below lines of intersection, location or amplitude of fiducial points, frequency content, the number of peaks, standard deviation, and the distance between peaks or any combination of the above.

In some implementations, processing or preprocessing can also be completed on the audio data, such as filtering or first or second derivatives or integrals. For example, analysis of generated acoustic data can include calculating a derivative of the cepstrum to determine a rate of change of the cepstrum signal. The derivative of the cepstrum can provide additional information that is used to detect conduit occlusions. The derivative of the cepstrum also allows the identification of alternate features from the same input.

Although the present disclosure describes the determination of an acoustic signature in the context of a cepstrum for the detection of conduit occlusions, it is contemplated that acoustic signatures may be detected by similar operations in one or more spectrums, time domain, auto-correlation, discrete cosine transform, cross-correlation, Mel-Frequency cepstral coefficients, linear predictive coding, wavelet decomposition, cepstrum, power cepstrum, and/or complex cepstrum.

In some implementations, the acoustic signal can be emitted into the tube connected to the user interface via an audio transducer, such as a speaker. Alternatively, or in addition, the acoustic signal can be emitted into the tube via a motor of the respiratory therapy device connected to the tube. For example, the motor of the respiratory therapy device can emit sound from the rotation of its fan blades as it generates air flow and forces air through the tube. The emitted sound can be the acoustic signal, or at least part of the acoustic signal.

In some implementations, the acoustic signal can be a sound audible to a human (e.g., an average human with average human hearing). A sound is audible to a human when the sound has an amplitude that is loud enough for detection by the human and when it has a frequency within the frequency range of human hearing, which is generally about 20 Hz to about 20,000 Hz. In some implementations, the acoustic signal can be a sound that is inaudible to a human (e.g., an average human with average human hearing). A sound is inaudible when the sound has an amplitude that is less than the lowest perceptible amplitude of a human. For example, in one or more implementations, the acoustic signal can be an ultrasonic sound that has a frequency that is higher than the highest perceptible frequency for human hearing.

In one or more implementations, the generated acoustic data can be generated from a plurality of acoustic reflections of a plurality of acoustic signals. The acoustic signals can be the same acoustic signal that is repeatedly emitted, or can be different acoustic signals that are emitted together, forming a single acoustic signal, or can be emitted separately. When there are multiple acoustic signals for multiple acoustic reflections, the generated acoustic data can be an average of the plurality of acoustic reflections of the plurality of the acoustic signals.

In some implementations, the generated acoustic data can include a primary reflection within the acoustic reflection of the acoustic signal. Additionally, or in the alternative, the generated acoustic data can include a secondary reflection within the acoustic reflection of the acoustic signal. Additionally, or in the alternative, the generated acoustic data can include a tertiary reflection within the acoustic reflection of the acoustic signal. The generated acoustic data can be analyzed from the primary, secondary, or tertiary reflections. For example, the acoustic signatures used to detect or identify a conduit occlusion can relate to the primary reflection within the generated acoustic data. Alternatively, the acoustic signatures used to detect or identify a conduit occlusion can relate to a reflection other than the primary reflection within the generated acoustic data, such as a secondary reflection, a tertiary reflection, etc. Alternatively, the acoustic signatures used to detect or identify a conduit occlusion can include combinations of reflections, such as the first and second reflections; the first and third reflections; the first, second and third reflections; the second and third reflections, etc.

In some implementations, the magnitudes of a cepstrum corresponding to a primary reflection may be larger than the magnitudes of a cepstrum corresponding to a secondary reflection. However, the shapes of the plots for the primary and secondary reflections may be similar. In one or more implementations, acoustic signatures in a secondary reflection can be used to confirm acoustic signatures in a primary reflection with respect to detecting a conduit occlusion of the user interface. In one or more implementations, the presence/absence of additional reflections (e.g., a secondary reflection and/or tertiary reflection) can be used as an acoustic signature for detecting a conduit occlusion of the user interface. In one or more implementations, the ratio of the secondary reflection (and/or tertiary reflection) with respect to the primary reflection can be used as an acoustic signature for detecting an occlusion of the user interface.

The respiratory therapy system analyzes the generated acoustic data to correlate the occlusion or non-occlusion features of the headgear conduit to one or more signatures within the generated acoustic data. The one or more signatures can include, for example, a maximum magnitude of a transform, a minimum magnitude of a transform, a standard deviation of a transform, a skewness of a transform, a kurtosis of a transform, a median of an absolute value of a transform, a sum of the absolute value of a transform, a sum of a positive area of a transform, a sum of a negative area of a transform, a fundamental frequency, an energy corresponding to the fundamental frequency, a mean energy, at least one resonant frequency of a combination of the conduit and the user interface, a change in the at least one resonant frequency, a number of peaks within a range, a peak prominence, distance between peaks, or a combination thereof. In one or more implementations, one or more of the signatures can be changes in one or more of the above aspects over time. The changes can be during a series of iterations of comparing determined acoustic signatures with baseline acoustic signatures, such as consecutive iterations over the course of a predetermined time period (e.g., ten seconds, twenty seconds, thirty seconds, one minute, a few minutes).

In some implementations, analyzing the generated acoustic data includes generating a spectrum of the generated acoustic data, such as calculating a transform (e.g., Discrete Fourier Transform DFT, or Discrete Cosine Transform DCT) of the generated acoustic data. Thereafter, analyzing the generated acoustic data can further include calculating a logarithm of the spectrum. Thereafter, analyzing the generated acoustic data can include calculating a cepstrum of the logarithm spectrum, such as calculating an inverse transform (e.g., iDFT or iDCT) of the logarithm spectrum. The calculated cepstrum can then be analyzed for one or more acoustic signatures that can be used to identify anomalies in a headgear conduit and determine if the identified anomaly relates to an occlusion.

In some implementations, the analysis of the generated acoustic data does not require analyzing all of the generated acoustic data. For example, analyzing the generated acoustic data can include selecting a segment of the above-described spectrum. After selecting the segment, the analysis can further include calculating a direct transform (e.g., DFT or DCT) of the segment of the spectrum. From the direct transform, the one or more features of the user interface can be correlated to one or more signatures within the Fourier transform of the segment to identify the category of the user interface. Alternatively, analyzing the generated acoustic data can include selecting a segment of the log spectrum of the generated acoustic data described above. From the log spectrum, the analysis can include calculating a transform of the log spectrum, such as a Fourier transform of the log spectrum. Alternatively, the analysis can include calculating an inverse transform of the log spectrum, or an inverse transform of the transform of the log spectrum.

In some implementations, a segment of the generated acoustic signal can be selected for analysis by trimming the generated acoustic data based on where within the generated acoustic data the one or more signatures that indicate where occlusions in the headgear user interface are generally located. This is generally based on a known location of the microphone and a known length of tube, at the end of which the headgear user interface and headgear conduits are located.

In some implementations, the acoustic signatures used to identify an occlusion in a headgear user interface can relate to the primary reflection within the generated acoustic data. Alternatively, the signatures used to categorize the user interface can relate to a reflection other than the primary reflection within the generated acoustic data, such as a secondary reflection, a tertiary reflection, etc. Alternatively, the signatures used to categorize the user interface can include combinations of reflections, such as the first and second reflections; the first and third reflections; the first, second and third reflections; the second and third reflections, etc.

According to some implementations, the determination of an occlusion in a headgear user interface can include the one or more signatures of the cepstrum, e.g., one or more peaks or troughs in the cepstrum, having a maximum magnitude larger than a threshold.

In some implementations, calculating the cepstrum can be achieved by processing the acoustic reflection in a series of stages: calculate the frequency spectrum of the acoustic reflection via a fast Fourier transform; take the natural logarithm of the absolute magnitude of this frequency spectrum; and perform the inverse Fourier transform and calculate the real part of the signal to produce a cepstrum. This process may be repeated over multiple time intervals and an average cepstrum may be estimated. The resulting cepstrum signal transforms the acoustic reflection into the quefrency domain which is analogous to separating reflections in time and/or distance. In one or more implementations, the cepstrum can be calculated using a fast Fourier transform of 4096 samples of the acoustic reflection with, optionally, 50% overlap of the samples. These 4096 samples represent about 0.2 seconds of generated acoustic data.

**In** some implementations, the analyzing of the generated acoustic data can include calculating a derivative of the cepstrum to determine a rate of change of the cepstrum signal. The derivative of the cepstrum can provide additional information that is determine if an identified anomaly relates to an occlusion.

**In** some implementations, the respiratory therapy system analyzing the generated acoustic data can include normalizing the generated acoustic data. Normalizing the generated acoustic data can account for confounding conditions. The confounding conditions can be, for example, microphone gain, breathing amplitude, therapy pressure, varying tube length, curled or stretched tube, different therapy pressures, ambient temperature, or a combination thereof.

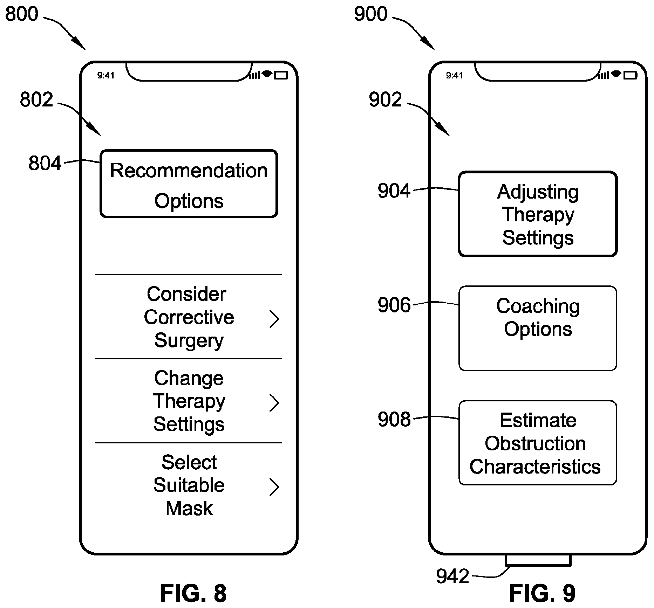

Referring to FIG. 8, a mobile phone 800 implements one or more of the components and functions discussed above in reference to the system 500 illustrated in FIG. 5. The mobile phone 800 includes a display 802 on which a recommendation 804 is provided, in response to determining the occurrence of and/or characterizing the physical obstruction P (illustrated in FIG. 5). For example, the recommendation 804 includes identifying a user as a candidate for corrective surgery. In another example, the recommendation 804 includes identifying a user as a candidate for alternative therapy. According to yet another example, the recommendation 804 identifies therapy settings and recommended changes to the therapy settings. According to yet another example, the recommendation is based on a user position, as determined by the motion sensor 516 (described above and illustrated in FIG. 5). According to yet another example, the recommendation 804 includes suggesting a sleep position for the user.

According to some implementations of the present disclosure, the recommendation 804 is further based on a user's medical history, in addition to physical characteristics of the physical obstruction P. For example, the recommendation 804 is further based on previous physical obstructions within the airway of the user.

According to some implementations of the present disclosure, the recommendation 804 includes identifying one or more masks suitable for use in a respiratory system (such as the PAP respiratory therapy system 120 described above in reference to FIGs. 1, 2A, and 2b). The mask includes, for example, a nasal mask, a nasal pillow mask, and a full-face mask.

Referring to FIG. 9, a mobile phone 900 implements one or more of the components and functions discussed above in reference to the system 500 illustrated in FIG. 5. The mobile phone 900 includes a display 902 on which various options are displayed, in response to determining the occurrence of the physical obstruction P (illustrated in FIG. 5). For example, the display 902 shows instructions for adjusting settings 904 of a therapy for treatment of apnea or hypopnea in real time. The settings 804 are automatically changed or are manually changed by a user or other person. In another example, the display 902 shows instructions for personalized coaching 906. Optionally, in addition to or instead of the display 902, the instructions are provided in audio form via a speaker 942 (which is similar or identical to the speaker 142 described above and illustrated in FIG. 1). The personalized coaching instructions 906 include, for example, changes, such as lifestyle changes, directed to obesity, sleeping position, or sleep hygiene. By "sleep hygiene," it is meant one or more of a recommended time to go to bed and/or to go to sleep, a recommended time to get out of bed and/or to wake, optimal lighting and/or temperature conditions of the sleep environment, etc.

According to some implementations of the present disclosure, the display 902 further displays instructions for estimating one or more physical characteristics of the physical obstruction P (illustrated in FIG. 5). For example, the physical characteristics include at least one of a size, shape, or location of the physical obstruction P.

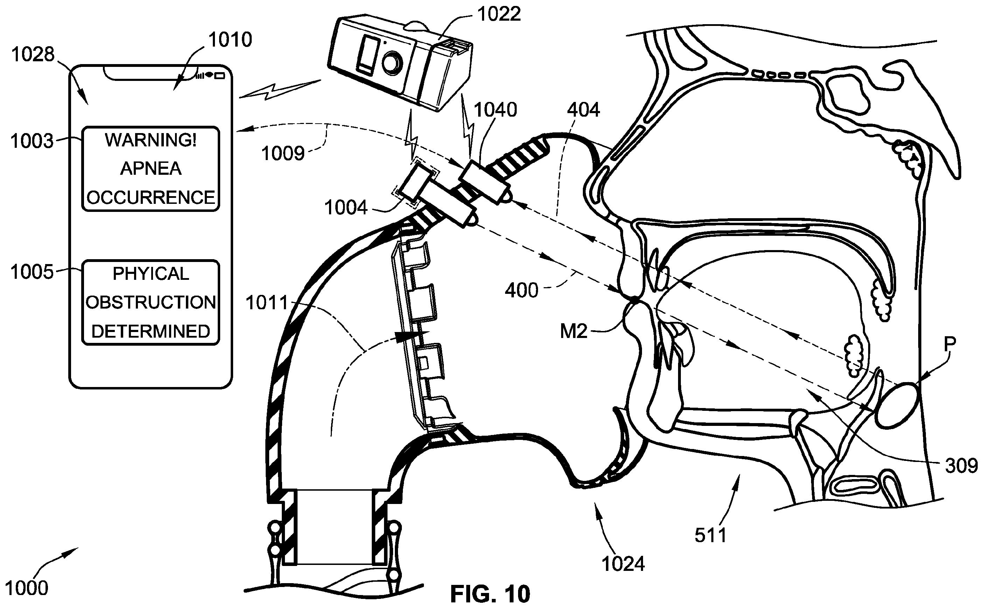

Referring to FIG. 10, a system 1000 is configured to determine both (a) the occurrence of an apnea event or hypopnea event and (b) characteristics of the physical obstruction P. According to some implementations, the system 1000 includes one or more components of the system 100 described above and illustrated in FIG. 1. The system 1000 includes a control system 1010, which may be in the form of a mobile phone 1010, or other electronic device, and which includes a display 1028. The mobile phone 1010 is communicatively coupled to a respiratory device 1022, a speaker 1004, and a microphone 1040. The respiratory device 1022 is similar or identical to the respiratory device 122 described above and illustrated in FIGs. 1, 2A, and 2B. The speaker 1004 and the microphone 1040 are fixed to a mask 1024, which is attached to mouth and nasal area of the user 511. According to one specific example, the speaker 1004 and the microphone 1040 are connected to a CPAP system (described above and illustrated in FIG. 1).

The respiratory device 1022 supplies pressurized air 1011 to the airway 309 of the user 511 during a sleep session. Flow data associated with the pressurized air 1011 is received and, based on the flow data, the system 1000 determines that an apnea event or a hypopnea event is occurring.

The speaker 1004 emits the acoustic signal 400 through the mask 1024, past the mouth M of the user 511, and into the airway 309 of the user 511. The acoustic signal 400 hits the physical obstruction P and reflects back in the form of the acoustic reflection 404. The acoustic reflection 404 is detected by the microphone 1040. Acoustic data 1009 associated with the acoustic reflection 404 is analyzed by the mobile phone 1010. In response to the analysis, the system 1000 the display 1028 shows an apnea indicia 1003 warning that an apnea occurrence has been determined (e.g., "WARNING! APNEA OCCURRENCE"). The display 1028 also shows an obstruction indicia 1005 indicating that the physical obstruction P has been characterized (e.g., "PHYSICAL OBSTRUCTION DETERMINED"). Optionally, assuming that the user 511 is asleep during the apnea occurrence, the system 1000 displays the apnea indicia 1003 and/or the obstruction indicia 1005 at a later time (e.g., the following morning) to the user 511 and/or a third party.

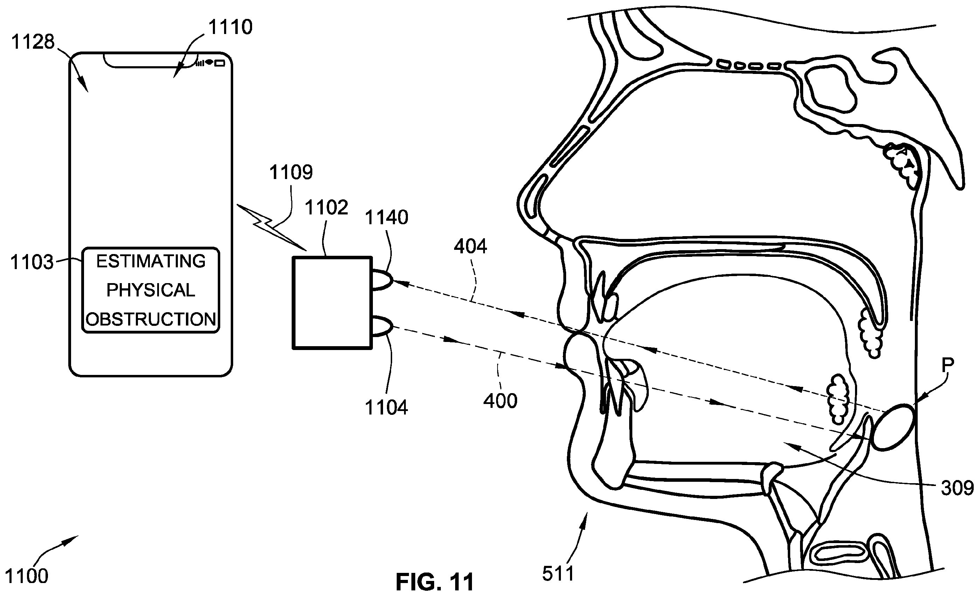

Referring to FIG. 11, a system 1100 is configured to determine characteristic of the physical obstruction P when an n apnea event or a hypopnea event is occurring. According to some implementations, the system 1100 includes one or more components described above and illustrated in FIG. 1. The system 1100 includes a control system 1110, which may be in the form of a mobile phone 1110. The mobile phone 1110 is communicatively coupled with an acoustic device 1102. The acoustic device 1102 is separate from or integrated with the mobile phone 1110. The acoustic device includes a speaker 1104 and a microphone 1140. Optionally, the speaker 1104 and the microphone 1140 are standard components of the mobile phone 1110 or are added as one or more after-market components. Optionally, if the acoustic device 1102 is integrated with the mobile phone 1110, a mounting bracket is configured for maintaining the mobile phone 1110 near the mouth of the user while the user is sleeping.

The mobile phone 1110 is configured to cause the speaker 1104 to emit the acoustic signal 400 into the airway 309 of the user 511. The acoustic signal 400 bounces off the physical obstruction O and reflects back in the form of the acoustic reflection 404. The microphone 1140 detects the acoustic reflection 404, and acoustic data 1109 associated with the acoustic reflection 404 is transmitted to and analyzed by the mobile phone 1110. A display 1128 of the mobile phone 1110 displays an indicia 1103 that the physical obstruction is being estimated.

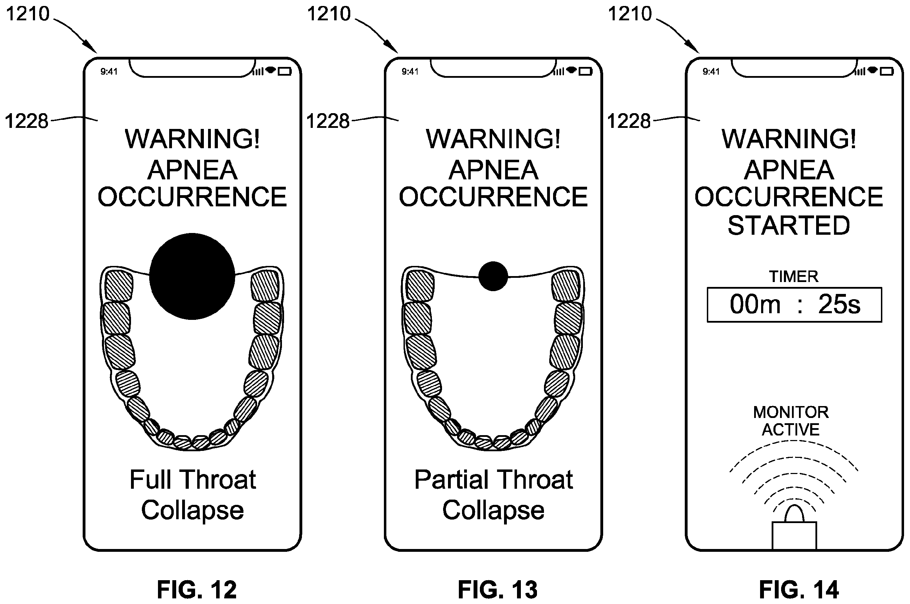

Referring to FIGs. 12-17, various representations are displayed on a display 1228 of a mobile phone 1210, which is configured to function in accordance with one or more embodiments described above. According to alternative embodiments, in accordance with the one or more embodiments described above, the mobile phone 1210 is a tablet, a computer screen, or a CPAP display. According to one exemplary embodiment, general recommendations are displayed to the user by detailed results are displayed to the doctor.

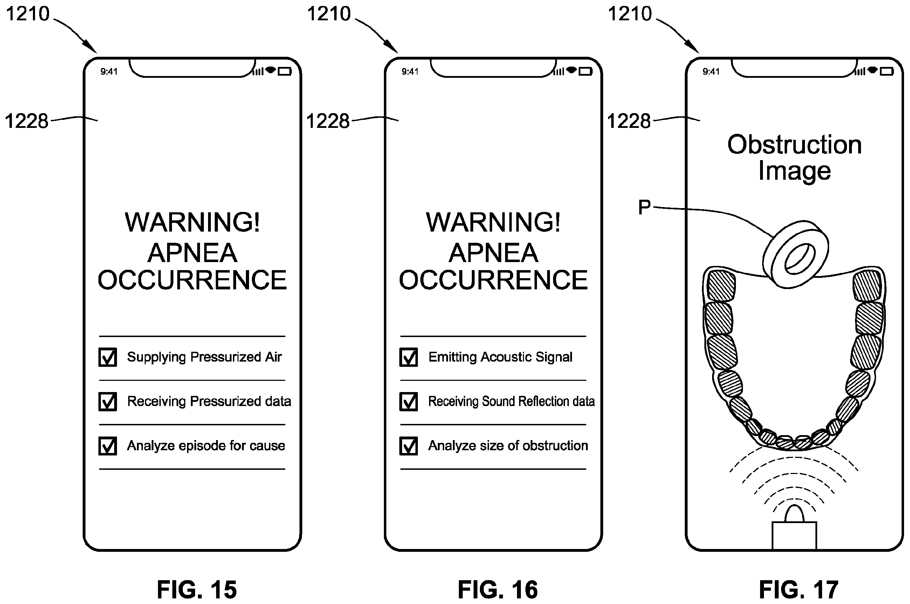

According one example, the mobile phone 1201 includes one or more components described above in reference to system 100 (illustrated in FIG. 1) and system 500 (illustrated in FIG. 5). In FIG. 12, the display 1228 illustrates a full throat collapse occurring during an apnea event. In FIG. 13, the display 1228 illustrates a partial throat collapses occurring during a hypopnea event. In FIG. 14, the display 1228 illustrates elapsed time since an apnea occurrence has started. In FIG. 15, the display 1228 illustrates a first set of settings, such as supplying pressurized air, receiving pressure data or flow data, and/or analyzing an apnea event for occurrence and/or cause. In FIG. 16, the display 1128 illustrates a second set of settings, such as emitting acoustic signal, receiving sound reflection data, and/or analyzing the size of the physical obstruction. In FIG. 17, the display 1128 illustrates an image or presentation of the physical obstruction P based on the analyzed sound reflection data.

## Claims

1. A system (500) comprising:
an acoustic device (502) for emitting an acoustic signal (400) and detecting an acoustic reflection (404);
a motion sensor (516) for detecting a user position:
a memory device (514) storing machine-readable instructions; and
a control system (510) including one or more processors communicatively coupled to the acoustic device and the motion sensor, the control system being configured to execute the machine-readable instructions to:
detect the acoustic reflection of the acoustic signal emitted by the acoustic device into an airway of the user, the acoustic reflection being caused by one or more physical features within the airway of the user,
analyze acoustic data associated with the acoustic reflection, and
characterize an occurrence of a physical obstruction in the airway of the user based, at least in part, on the analyzed acoustic data, the characterization being indicative of an apnea event or a hypopnea event in the user, wherein the apnea event comprises an obstructive apnea event, a central apnea event or a mixed apnea event, the characterization further distinguishing between the occurrence of the obstructive apnea event, the central apnea event, the mixed apnea event or the hypopnea event in the user, the characterization further determining a site location, a site size, and/or a site shape of the physical obstruction in the airway of the user,
wherein the user position is detected based on motion data from the motion sensor; and
provide a recommendation based on the physical obstruction and on the user position.

2. The system of claim 1, wherein the apnea event is the central apnea event, wherein the control system is further configured to execute the machine-readable instructions to characterize the occurrence based, at least in part, on respiration data indicative of a breathing pattern of the user, and, optionally, wherein the respiration data is derived, at least in part, from (i) flow and/or pressure data from a respiratory therapy system, (ii) passive acoustic data from a passive acoustic sensor, (iii) motion data from the motion sensor, or (iv) any combination of (i) to (iii).

3. The method of any one of claims 1 to 2, wherein characterization of the occurrence of the physical obstruction in the airway of the user indicates the absence of the physical obstruction.

4. The system of any one of claims 1 to 2, wherein the control system is further configured to execute the machine-readable instructions determine a duration of the physical obstruction within the airway of the user; and/or
wherein the control system is further configured to execute the machine-readable instructions to emit the acoustic signal at specific monitoring times after detection of the apnea event or the hypopnea event; and/or
wherein the control system is further configured to execute the machine-readable instructions to emit the acoustic signal intermittently or continuously; and/or
wherein the control system is configured to execute the machine-readable instructions to characterize the occurrence of the physical obstruction based solely on the analyzed acoustic data.

5. The system of any one of claims 1 to 4, wherein the control system is further configured to execute the machine-readable instructions to monitor the acoustic reflection for a time period sufficient to capture time of flight to two or more sites of the physical obstruction, the physical obstruction having multiple sites extending through the airway of the user; and, optionally, wherein a minimum time period for monitoring the acoustic reflection is at least about 0.15 seconds from when the acoustic signal is emitted, or at least about 4 seconds from when the acoustic signal is emitted.

6. The system of any one of claims 1 to 5, wherein the control system is further configured to execute the machine-readable instructions to sample the acoustic data during a portion or all of the apnea event or the hypopnea event; and/or
wherein the control system is further configured to execute the machine-readable instructions to characterize the occurrence of the physical obstruction within about 0.25 seconds from start of the apnea event or the hypopnea event; and/or
wherein the control system is further configured to execute the machine-readable instructions to characterize the occurrence of the physical obstruction after approximately 4-6 seconds of a beginning of the apnea event or the hypopnea event, the beginning of the apnea event or the hypopnea event being independently determined; and/or
further comprising determining the central apnea via one or more of (i) a respiratory therapy system, (ii) an acoustic sensor, and (iii) the motion sensor;
optionally, wherein the characterization indicates the absence of the physical obstruction; or
wherein the independently determining is based on (i) flow data, (ii) pressure data, or (iii) both (i) and (ii).

7. The system of any one of claims 1 to 6, wherein the acoustic data includes a plurality of audio samples, wherein the audio samples correspond to a plurality of acoustic reflections received over a period of time, optionally, wherein the control system is further configured to execute the machine-readable instructions to average results from the plurality of audio samples; and/or
wherein the control system is further configured to execute the machine-readable instructions to determine if the airway is in a partially collapsed state, the partially collapsed state being indicative of the hypopnea event; and/or
wherein the control system is further configured to execute the machine-readable instructions to determine if the airway is in a fully collapsed state, the fully collapsed state being indicative of the apnea event; and/or
wherein the acoustic data is indicative of a first shape of the physical obstruction if the hypopnea event occurs, the acoustic data being indicative of a second shape if the apnea event occurs,
optionally, wherein the first shape is in the form of a first amplitude and the second shape is in the form of a second amplitude, the first amplitude being different than the second amplitude.

8. The system of claim 1, wherein (i) the recommendation identifies the user as a candidate for corrective surgery; (ii) the recommendation identifies the user as a candidate for alternative therapy, optionally, wherein the alternative therapy is achieved via a mandibular repositioning device (MRD); (iii) the recommendation identifies therapy settings or a modification of current therapy settings; (iv) the recommendation is further based on user's medical history; (v) the recommendation is further based on previous physical obstructions within the airway of the user; (vi) the recommendation includes identifying one or more user interfaces suitable for use in a respiratory therapy system for treatment of apnea or hypopnea, optionally, wherein the one or more user interfaces are selected from a group comprising or consisting of a nasal mask, a nasal pillow mask, and a full-face mask.

9. The system of any one of claims 1 to 8, wherein the control system is further configured to execute the machine-readable instructions to adjust settings of a therapy for treatment of apnea or hypopnea in real time; and/or
wherein the control system is further configured to execute the machine-readable instructions to enable instructions for personalized coaching, optionally via a display or speaker, optionally, wherein the instructions include suggested changes directed to obesity, sleeping position, or sleep hygiene.

10. The system of any one of claims 1 to 9, wherein the control system is further configured to execute the machine-readable instructions to estimate one or more physical characteristics of the physical obstruction, optionally, wherein the one or more physical characteristics include at least one physical characteristic selected from a group consisting of a size, a shape, or a location of the physical obstruction; and/or
wherein the one or more physical characteristics are estimated based on a change in acoustic impedance; and/or
wherein the one or more physical characteristics are estimated based on analyzing the acoustic reflection that occurs from the physical obstruction within the airway of the user;
wherein the one or more physical characteristics are estimated based on a comparison between (a) one or more acoustic signals reflecting at a point M before an entry into the airway of the user and (b) one or more acoustic signals reflecting at a point P after the entry into the airway of the user; and/or
wherein the one or more physical characteristics are estimated based on a distance travelled by one or more acoustic signals emitted into the airway of the user past an entry point, wherein the entry point is a mouth area or a nose area of the user, the airway being in a throat area of the user.

11. The system of any one of claims 1 to 10, wherein the characterization is further based on a physical measurement of at least one of the mouth area, the nose area, and the throat area; and/or
wherein the one or more physical characteristics are estimated based on a signal morphology of the acoustic reflection, optionally, wherein the signal morphology includes an acoustic shape of an acoustic wave of the acoustic reflection; and/or
wherein the one or more physical characteristics are estimated based in part on one or more physical or demographic characteristics of the user, optionally, wherein the one or more user characteristics is selected from a group comprising or consisting of age, sex, weight, mouth size, neck size, or a type of user interface currently used by the user, or any combination thereof; and/or
wherein the control system is further configured to execute the machine-readable instructions to identify a location of a body organ of the user, optionally, wherein the body organ is a lung; and/or
wherein the acoustic device includes a motor, wherein the motor is configured to emit the acoustic signal; and/or
wherein the acoustic device includes a speaker, the speaker emitting the acoustic signal, optionally, wherein the speaker generates the acoustic signal in the form of a sound, the sound being selected from a group comprising or consisting of one or more of a standard sound, a custom sound, an inaudible frequency, a white noise sound, a broad band impulse, a continuous sinusoidal waveform, or any combination thereof; and/or
wherein the acoustic signal is in the form of a sound selected from a group consisting of an audible sound and an ultrasonic sound; and/or
wherein the control system is further configured to execute the machine-readable instructions to obtain a cepstrum of the acoustic reflection, optionally, wherein the cepstrum identifies a distance associated with the acoustic reflection, the distance showing a location of the physical obstruction within the airway of the user, further optionally, wherein the cepstrum identifying a distance between a user interface positioned on the user's face and the physical obstruction.

12. The system of any one of claims 1 to 11, wherein the user position includes one or more of a user location and a user orientation; and/or
wherein the motion sensor is selected from a group consisting of a camera, a bed sensor, an accelerometer, a sonar sensor, and a radio frequency sensor such as an impulse-radio ultra wideband (IR-UWB) sensor.

13. The system of any one of claims 1 to 12, further comprising a respiratory therapy system for treating obstructive sleep apnea, optionally wherein the respiratory therapy system is capable of independently determining an occurrence of the apnea event or the hypopnea event is based on (i) flow data, (ii) pressure data, or (iii) both (i) and (ii), detected by the respiratory therapy system; and/or
wherein the acoustic device includes a microphone for detecting the acoustic reflection of the acoustic signal.

14. The system of claim 1, further comprising:
a respiratory therapy system;
the acoustic device being associated with the respiratory therapy system; and
the control system being further configured to execute the machine-readable instructions to:
supply pressurized air, via the respiratory therapy system, to the airway of the user during a sleep session;
receive flow data and/or pressure data associated with the pressurized air; and
determine, based on the flow data and/or pressure data, occurrence of the apnea event or the hypopnea event;
wherein, optionally, the respiratory therapy system is a positive airway pressure (PAP) system.

## Patentansprüche

1. System (500), umfassend:
eine akustische Vorrichtung (502) zum Aussenden eines akustischen Signals (400) und zum Erfassen einer akustischen Reflexion (404);
ein Bewegungssensor (516) zum Erfassen einer Benutzerposition;
eine Speichervorrichtung (514), die maschinenlesbare Anweisungen speichert; und
ein Steuerungssystem (510), das einen oder mehrere Prozessoren beinhaltet, die kommunikativ mit der akustischen Vorrichtung und dem Bewegungssensor gekoppelt sind, wobei das Steuerungssystem konfiguriert ist, um die maschinenlesbaren Anweisungen auszuführen, um:
die akustische Reflexion des von der akustischen Vorrichtung ausgesendeten akustischen Signals in die Atemwege des Benutzers zu erfassen, wobei die akustische Reflexion durch ein oder mehrere physikalische Merkmale innerhalb der Atemwege des Benutzers bewirkt wird,
akustische Daten zu analysieren, die der akustischen Reflexion zugeordnet sind, und
ein Auftreten einer physischen Obstruktion in den Atemwegen des Benutzers mindestens teilweise basierend auf den analysierten akustischen Daten zu charakterisieren, wobei die Charakterisierung auf ein Apnoe-Ereignis oder ein Hypopnoe-Ereignis beim Benutzer hinweist, wobei das Apnoe-Ereignis ein obstruktives Apnoe-Ereignis, ein zentrales Apnoe-Ereignis oder ein gemischtes Apnoe-Ereignis umfasst, wobei die Charakterisierung weiter zwischen dem Auftreten des obstruktiven Apnoe-Ereignisses, des zentralen Apnoe-Ereignisses, des gemischten Apnoe-Ereignisses oder des Hypopnoe-Ereignisses beim Benutzer unterscheidet, wobei die Charakterisierung weiter eine Ortslokalisierung, eine Ortsgröße und/oder eine Ortsgestalt der physischen Obstruktion in den Atemwegen des Benutzers bestimmt,
wobei die Benutzerposition basierend auf Bewegungsdaten vom Bewegungssensor erfasst wird; und
eine Empfehlung basierend auf der physischen Obstruktion und der Benutzerposition bereitzustellen.

2. System nach Anspruch 1, wobei das Apnoe-Ereignis das zentrale Apnoe-Ereignis ist, wobei das Steuerungssystem weiter konfiguriert ist, um die maschinenlesbaren Anweisungen auszuführen, um das Auftreten mindestens teilweise basierend auf Atmungsdaten zu charakterisieren, die auf ein Atemmuster des Benutzers hinweisen, und wobei die Atmungsdaten optional mindestens teilweise aus (i) Fluss und/oder Druckdaten aus einem Beatmungstherapiesystem, (ii) passiven akustischen Daten von einem passiven akustischen Sensor, (iii) Bewegungsdaten vom Bewegungssensor oder (iv) einer beliebigen Kombination von (i) bis (iii) abgeleitet werden.

3. Verfahren nach einem der Ansprüche 1 oder 2, wobei Charakterisieren des Auftretens der physischen Obstruktion in den Atemwegen des Benutzers die Abwesenheit der physischen Obstruktion angibt.

4. System nach einem der Ansprüche 1 bis 2, wobei das Steuerungssystem weiter konfiguriert ist, um die maschinenlesbaren Anweisungen auszuführen, um eine Dauer der physischen Obstruktion in den Atemwegen des Benutzers zu bestimmen; und/oder
wobei das Steuerungssystem weiter konfiguriert ist, um die maschinenlesbaren Anweisungen auszuführen, um das akustische Signal zu bestimmten Überwachungszeitpunkten nach Erfassen des Apnoe- oder Hypopnoe-Ereignisses auszusenden; und/oder
wobei das Steuerungssystem weiter konfiguriert ist, um die maschinenlesbaren Anweisungen auszuführen, um das akustische Signal intermittierend oder kontinuierlich auszusenden; und/oder
wobei das Steuerungssystem konfiguriert ist, um die maschinenlesbaren Anweisungen auszuführen, um das Auftreten der physischen Obstruktion ausschließlich basierend auf den analysierten akustischen Daten zu charakterisieren.

5. System nach einem der Ansprüche 1 bis 4, wobei das Steuerungssystem weiter konfiguriert ist, um die maschinenlesbaren Anweisungen auszuführen, um die akustische Reflexion über einen Zeitraum hinweg zu überwachen, der ausreicht, um die Lichtlaufzeit zu zwei oder mehr Orten der physischen Obstruktion festzuhalten, wobei die physische Obstruktion mehrere Orte aufweist, die sich durch die Atemwege des Benutzers erstrecken; und wobei ein Mindestzeitraum zur Überwachung der akustischen Reflexion optional mindestens etwa 0,15 Sekunden ab dem Zeitpunkt der Aussendung des akustischen Signals oder mindestens etwa 4 Sekunden ab dem Zeitpunkt der Aussendung des akustischen Signals beträgt.

6. System nach einem der Ansprüche 1 bis 5, wobei das Steuerungssystem weiter konfiguriert ist, um die maschinenlesbaren Anweisungen auszuführen, um die akustischen Daten während eines Abschnitts oder des gesamten Apnoe- oder Hypopnoe-Ereignisses abzutasten; und/oder
wobei das Steuerungssystem weiter konfiguriert ist, um die maschinenlesbaren Anweisungen auszuführen, um das Auftreten der physischen Obstruktion innerhalb von etwa 0,25 Sekunden nach Beginn des Apnoe- oder Hypopnoe-Ereignisses zu charakterisieren; und/oder
wobei das Steuerungssystem weiter konfiguriert ist, um die maschinenlesbaren Anweisungen auszuführen, um das Auftreten der physischen Obstruktion nach etwa 4-6 Sekunden nach Beginn des Apnoe-Ereignisses oder Hypopnoe-Ereignisses zu charakterisieren, wobei der Beginn des Apnoe- oder Hypopnoe-Ereignisses unabhängig bestimmt wird; und/oder
weiter umfassend Bestimmen der zentralen Apnoe über eines oder mehr von (i) einem Beatmungstherapiesystem, (ii) einem akustischen Sensor und (iii) dem Bewegungssensor;
wobei die Charakterisierung optional die Abwesenheit der physischen Obstruktion angibt; oder
wobei das unabhängig Bestimmen auf (i) Flussdaten, (ii) Druckdaten oder (iii) beiden (i) und (ii) basiert.

7. System nach einem der Ansprüche 1 bis 6, wobei die akustischen Daten eine Vielzahl von Audioproben beinhalten, wobei die Audioproben einer Vielzahl von akustischen Reflexionen entsprechen, die über einen Zeitraum empfangen werden, wobei das Steuerungssystem optional weiter konfiguriert ist, um die maschinenlesbaren Anweisungen auszuführen, um die Ergebnisse der Vielzahl von Audioproben zu mitteln; und/oder
wobei das Steuerungssystem weiter konfiguriert ist, um die maschinenlesbaren Anweisungen auszuführen, um zu bestimmen, ob sich die Atemwege in einem teilweise kollabierten Zustand befinden, wobei der teilweise kollabierte Zustand auf das Hypopnoe-Ereignis hinweist; und/oder
wobei das Steuerungssystem weiter konfiguriert ist, um die maschinenlesbaren Anweisungen auszuführen, um zu bestimmen, ob sich die Atemwege in einem vollständig kollabierten Zustand befinden, wobei der vollständig kollabierte Zustand auf das Hypopnoe-Ereignis hinweist; und/oder
wobei die akustischen Daten auf eine erste Gestalt der physischen Obstruktion hinweisen, wenn das Hypopnoe-Ereignis eintritt, wobei die akustischen Daten auf eine zweite Gestalt hinweisen, wenn das Apnoe-Ereignis eintritt,
wobei die erste Gestalt optional die Gestalt einer ersten Amplitude und die zweite Gestalt die Gestalt einer zweiten Amplitude aufweist, wobei sich die erste Amplitude von der zweiten Amplitude unterscheidet.

8. System nach Anspruch 1, wobei (i) die Empfehlung den Benutzer als Kandidaten für eine Korrekturoperation identifiziert; (ii) die Empfehlung den Benutzer als Kandidaten für eine alternative Therapie identifiziert, wobei die alternative Therapie optional mittels einer mandibulären Neupositionierungsvorrichtung (MRD) erreicht wird; (iii) die Empfehlung Therapieeinstellungen oder eine Modifikation aktueller Therapieeinstellungen identifiziert; (iv) die Empfehlung weiter auf der Krankengeschichte des Benutzers basiert; (v) die Empfehlung weiter auf früheren physischen Obstruktionen der Atemwege des Benutzers basiert; (vi) die Empfehlung die Identifizierung einer oder mehrerer Benutzerschnittstellen beinhaltet, die für die Verwendung in einem Beatmungstherapiesystem zur Behandlung von Apnoe oder Hypopnoe geeignet sind, wobei die eine oder mehreren Benutzerschnittstellen optional aus einer Gruppe ausgewählt sind, die eine Nasenmaske, eine Nasenpolstermaske und eine Vollgesichtsmaske umfasst oder daraus besteht.

9. System nach einem der Ansprüche 1 bis 8, wobei das Steuerungssystem weiter konfiguriert ist, um die maschinenlesbaren Anweisungen auszuführen, um die Einstellungen einer Therapie zur Behandlung von Apnoe oder Hypopnoe in Echtzeit anzupassen; und/oder
wobei das Steuerungssystem weiter konfiguriert ist, um die maschinenlesbaren Anweisungen auszuführen, um Anweisungen für personalisiertes Coaching optional über ein Display oder einen Lautsprecher zu ermöglichen, wobei die Anweisungen optional vorgeschlagene Änderungen beinhalten, die auf Fettleibigkeit, Schlafposition oder Schlafhygiene gerichtet sind.

10. System nach einem der Ansprüche 1 bis 9, wobei das Steuerungssystem weiter konfiguriert ist, um die maschinenlesbaren Anweisungen auszuführen, um eine oder mehrere physischen Eigenschaften der physischen Obstruktion zu schätzen, wobei die eine oder mehrere physische Eigenschaften mindestens eine physische Eigenschaft beinhalten, die aus einer Gruppe bestehend aus Größe, Gestalt oder Lage der physischen Obstruktion ausgewählt ist; und/oder
wobei die eine oder mehrere physische Eigenschaften basierend auf einer Änderung der akustischen Impedanz geschätzt werden; und/oder
wobei die eine oder mehrere physische Eigenschaften basierend auf Analysieren der akustischen Reflexion geschätzt werden, die von der physischen Obstruktion in den Atemwegen des Benutzers ausgeht;
wobei die eine oder mehrere physische Eigenschaften basierend auf einem Vergleich zwischen (a) einem oder mehreren akustischen Signalen, die an einem Punkt M vor dem Eintritt in die Atemwege des Benutzers reflektiert werden, und (b) einem oder mehreren akustischen Signalen, die an einem Punkt P nach dem Eintritt in die Atemwege des Benutzers reflektiert werden, geschätzt werden; und/oder
wobei die eine oder mehrere physische Eigenschaften basierend auf einer Entfernung geschätzt werden, die von einem oder mehreren akustischen Signalen zurückgelegt werden, die in die Atemwege des Benutzers nach einem Eintrittspunkt ausgesendet werden, wobei der Eintrittspunkt ein Mundbereich oder Nasenbereich des Benutzers ist und sich die Atemwege im Rachenbereich des Benutzers befinden.

11. System nach einem der Ansprüche 1 bis 10, wobei die Charakterisierung weiter auf einer physischen Messung mindestens eines vom Mundbereich, Nasenbereich und Rachenbereich basiert; und/oder
wobei die eine oder mehrere physische Eigenschaften basierend auf einer Signalmorphologie der akustischen Reflexion geschätzt werden, wobei die Signalmorphologie optional eine akustische Gestalt einer akustischen Welle der akustischen Reflexion beinhaltet; und/oder
wobei die eine oder mehrere physische Eigenschaften teilweise basierend auf einer oder mehreren physischen oder demografischen Eigenschaften des Benutzers geschätzt werden, wobei die eine oder mehrere Benutzereigenschaften aus einer Gruppe ausgewählt werden, die Alter, Geschlecht, Gewicht, Mundgröße, Halsgröße oder eine Art von Benutzerschnittstelle umfasst, die der Benutzer derzeit verwendet, oder eine beliebige Kombination davon; und/oder
wobei das Steuerungssystem weiter konfiguriert ist, um die maschinenlesbaren Anweisungen auszuführen, um einen Standort eines Körperorgans des Benutzers zu identifizieren, wobei das Körperorgan optional eine Lunge ist; und/oder
wobei die akustische Vorrichtung einen Motor beinhaltet, der konfiguriert ist, um das akustische Signal auszusenden; und/oder
wobei die akustische Vorrichtung einen Lautsprecher beinhaltet, der das akustische Signal aussendet, wobei der Lautsprecher das akustische Signal optional in Form eines Tons erzeugt, wobei der Ton aus einer Gruppe ausgewählt ist, die eines oder mehrere von einem Standardton, einem benutzerdefinierten Ton, einer unhörbaren Frequenz, einem weißen Rauschen, einem Breitbandimpuls, einer kontinuierlichen Sinuswelle oder einer beliebigen Kombination davon umfasst oder daraus besteht; und/oder
wobei das akustische Signal in Form eines Tons vorliegt, der aus einer Gruppe bestehend aus einem hörbaren Ton und einem Ultraschallton ausgewählt ist; und/oder
wobei das Steuerungssystem weiter konfiguriert ist, um die maschinenlesbaren Anweisungen auszuführen, um ein Cepstrum der akustischen Reflexion zu erhalten, wobei das Cepstrum optional einen der akustischen Reflexion zugeordneten Abstand identifiziert, wobei der Abstand eine Stelle der physischen Obstruktion in den Atemwegen des Benutzers zeigt, wobei das Cepstrum weiter optional einen Abstand zwischen einer auf dem Gesicht des Benutzers positionierten Benutzerschnittstelle und der physischen Obstruktion identifiziert.

12. System nach einem der Ansprüche 1 bis 11, wobei die Benutzerposition eines oder mehrere von einer den Benutzerstelle und einer Benutzerausrichtung beinhaltet; und/oder
wobei der Bewegungssensor aus einer Gruppe bestehend aus einer Kamera, einem Bettsensor, einem Beschleunigungsmesser, einem Sonarsensor und einem Hochfrequenzsensor wie einem Impulsradio-Ultrabreitbandsensor (IR-UWB) ausgewählt ist.

13. System nach einem der Ansprüche 1 bis 12, weiter umfassend ein Beatmungstherapiesystem zur Behandlung der obstruktiven Schlafapnoe, wobei das Beatmungstherapiesystem optional in der Lage ist, unabhängig ein Auftreten eines Apnoe-Ereignisses oder Hypopnoe-Ereignisses basierend auf (i) Flussdaten, (ii) Druckdaten oder (iii) beiden (i) und (ii) zu bestimmen, die vom Beatmungstherapiesystem erfasst werden; und/oder
wobei die akustische Vorrichtung ein Mikrofon zur Erfassung der akustischen Reflexion des akustischen Signals beinhaltet.

14. System nach Anspruch 1, weiter umfassend:
ein Beatmungstherapiesystem;
die akustische Vorrichtung, die dem Beatmungstherapiesystem zugeordnet ist; und
das Steuerungssystem weiter konfiguriert ist, um die maschinenlesbaren Anweisungen auszuführen, um:
Druckluft über das Beatmungstherapiesystem in die Atemwege des Benutzers während einer Schlafsitzung zuzuführen;
der Druckluft zugeordnete Flussdaten und/oder Druckdaten zu empfangen; und basierend auf den Flussdaten und/oder Druckdaten Auftreten eines Apnoe- oder Hypopnoe-Ereignisses zu bestimmen;
wobei das Beatmungstherapiesystem optional ein positiver Atemwegsdruck-(PAP-) System ist.

## Revendications

1. Système (500) comprenant :
un dispositif acoustique (502) pour émettre un signal acoustique (400) et détecter une réflexion acoustique (404) ;
un capteur de mouvement (516) pour détecter la position d'un utilisateur ;
un dispositif de mémoire (514) stockant des instructions lisibles par machine ; et
un système de commande (510) incluant un ou plusieurs processeurs connectés de manière communicative au dispositif acoustique et au capteur de mouvement, le système de commande étant configuré pour exécuter les instructions lisibles par machine pour :
détecter la réflexion acoustique du signal acoustique émis par le dispositif acoustique dans les voies respiratoires de l'utilisateur, la réflexion acoustique étant causée par une ou plusieurs caractéristiques physiques des voies respiratoires de l'utilisateur.
analyser des données acoustiques associées à la réflexion acoustique, et
caractériser une occurrence d'une obstruction physique des voies respiratoires de l'utilisateur sur la base, au moins en partie, des données acoustiques analysées, la caractérisation étant indicative d'un épisode d'apnée ou d'hypopnée chez l'utilisateur, dans lequel l'événement d'apnée comprend un événement d'apnée obstructive, un événement d'apnée centrale ou un événement d'apnée mixte, la caractérisation faisant en outre la distinction entre l'occurrence de l'événement d'apnée obstructive, de l'événement d'apnée centrale, de l'événement d'apnée mixte ou de l'événement d'hypopnée chez l'utilisateur, la caractérisation déterminant en outre une localisation du site, une taille du site et/ou une forme du site de l'obstruction physique dans les voies respiratoires de l'utilisateur,
dans lequel la position de l'utilisateur est détectée sur la base de données de mouvement provenant du capteur de mouvement ; et
formuler une recommandation sur la base de l'obstruction physique et de la position de l'utilisateur.

2. Système selon la revendication 1, dans lequel l'événement d'apnée est l'événement d'apnée centrale, dans lequel le système de commande est en outre configuré pour exécuter des instructions lisibles par machine pour caractériser l'occurrence sur la base, au moins en partie, de données de respiration indicatives d'un profil respiratoire de l'utilisateur, et, optionnellement, dans lequel les données de respiration sont dérivées, au moins en partie, (i) de données de pression et/ou de débit provenant d'un système de thérapie respiratoire, (ii) de données acoustiques passives provenant d'un capteur acoustique passif, (iii) de données de mouvement provenant d'un capteur de mouvement, ou (iv) toute combinaison de (i) à (iii).

3. Procédé selon l'une quelconque des revendications 1 à 2, dans lequel la caractérisation de l'occurrence de l'obstruction physique dans les voies respiratoires de l'utilisateur indique l'absence de l'obstruction physique.

4. Système selon l'une quelconque des revendications 1 à 2, dans lequel le système de commande est en outre configuré pour exécuter les instructions lisibles par machine pour déterminer une durée de l'obstruction physique dans les voies respiratoires de l'utilisateur ; et/ou
dans lequel le système de commande est en outre configuré pour exécuter les instructions lisibles par machine pour émettre le signal acoustique à des moments de surveillance spécifiques après la détection de l'événement d'apnée ou de l'événement d'hypopnée ; et/ou
dans lequel le système de commande est en outre configuré pour exécuter les instructions lisibles par machine pour émettre le signal acoustique de manière intermittente ou continue ; et/ou
dans lequel le système de commande est configuré pour exécuter les instructions lisibles par machine pour caractériser l'occurrence de l'obstruction physique uniquement sur la base des données acoustiques analysées.

5. Système selon l'une quelconque des revendications 1 à 4, dans lequel le système de commande est en outre configuré pour exécuter les instructions lisibles par machine pour surveiller la réflexion acoustique pendant une période de temps suffisante pour capturer le temps de vol vers deux sites ou plus de l'obstruction physique, l'obstruction physique présentant plusieurs sites s'étendant à travers les voies respiratoires de l'utilisateur ; et, optionnellement, dans lequel une période de temps minimale pour la surveillance de la réflexion acoustique est d'au moins environ 0,15 seconde à compter du moment où le signal acoustique est émis, ou d'au moins environ 4 secondes à compter du moment où le signal acoustique est émis.

6. Système selon l'une quelconque des revendications 1 à 5, dans lequel le système de commande est en outre configuré pour exécuter les instructions lisibles par machine pour échantillonner les données acoustiques pendant une partie ou la totalité de l'événement d'apnée ou de l'événement d'hypopnée ; et/ou
dans lequel le système de commande est en outre configuré pour exécuter les instructions lisibles par machine pour caractériser l'occurrence de l'obstruction physique dans un délai d'environ 0,25 seconde à partir d'un début de l'événement d'apnée ou de l'événement d'hypopnée ; et/ou
dans lequel le système de commande est en outre configuré pour exécuter les instructions lisibles par machine pour caractériser l'occurrence de l'obstruction physique environ 4 à 6 secondes après un commencement de l'événement d'apnée ou de l'événement d'hypopnée, le commencement de l'événement d'apnée ou de l'événement d'hypopnée étant déterminé indépendamment ; et/ou
comprenant en outre la détermination de l'apnée centrale via un ou plusieurs parmi (i) un système de thérapie respiratoire, (ii) un capteur acoustique et (iii) le capteur de mouvement ;
optionnellement, dans lequel la caractérisation indique l'absence de l'obstruction physique ; ou
dans lequel la détermination indépendante est basée sur (i) des données de débit, (ii) des données de pression, ou (iii) à la fois (i) et (ii).

7. Système selon l'une quelconque des revendications 1 à 6, dans lequel les données acoustiques incluent une pluralité d'échantillons audio, dans lequel les échantillons audio correspondent à une pluralité de réflexions acoustiques reçues sur une période de temps, optionnellement, dans lequel le système de commande est en outre configuré pour exécuter les instructions lisibles par machine pour faire la moyenne de résultats provenant de la pluralité d'échantillons audio ; et/ou
dans lequel le système de commande est en outre configuré pour exécuter les instructions lisibles par machine pour déterminer si les voies respiratoires sont dans un état partiellement collabé, l'état partiellement collabé étant indicatif de l'événement d'hypopnée ; et/ou
dans lequel le système de commande est en outre configuré pour exécuter les instructions lisibles par machine pour déterminer si les voies respiratoires sont dans un état totalement collabé, l'état totalement collabé étant indicatif de l'événement d'apnée ; et/ou
dans lequel les données acoustiques sont indicatives d'une première forme de l'obstruction physique si l'événement d'hypopnée se produit, les données acoustiques étant indicatives d'une seconde forme si l'événement d'apnée se produit,
optionnellement, dans lequel la première forme est sous la forme d'une première amplitude et la seconde forme est sous la forme d'une seconde amplitude, la première amplitude étant différente de la seconde amplitude.

8. Système selon la revendication 1, dans lequel : (i) la recommandation identifie l'utilisateur comme candidat à une chirurgie corrective ; (ii) la recommandation identifie l'utilisateur comme candidat à une thérapie alternative, optionnellement, dans lequel la thérapie alternative est accomplie au moyen d'un dispositif de repositionnement mandibulaire (DRM) ; (iii) la recommandation identifie des paramètres de thérapie ou une modification de paramètres de thérapie actuels ; (iv) la recommandation est en outre basée sur des antécédents médicaux de l'utilisateur ; (v) la recommandation est en outre basée sur des obstructions physiques précédentes des voies respiratoires de l'utilisateur ; (vi) la recommandation inclut l'identification d'une ou plusieurs interfaces utilisateur adaptées à une utilisation dans un système de thérapie respiratoire pour le traitement de l'apnée ou de l'hypopnée, optionnellement, dans lequel les une ou plusieurs interfaces utilisateur sont sélectionnées parmi un groupe comprenant ou consistant en un masque nasal, un masque à coussinets nasaux et un masque facial complet.

9. Système selon l'une quelconque des revendications 1 à 8, dans lequel le système de commande est en outre configuré pour exécuter les instructions lisibles par machine pour ajuster des paramètres d'une thérapie pour le traitement de l'apnée ou de l'hypopnée en temps réel ; et/ou
dans lequel le système de commande est en outre configuré pour exécuter les instructions lisibles par machine pour permettre des instructions pour un coaching personnalisé, optionnellement via un écran ou un haut-parleur, dans lequel les instructions incluent des modifications suggérées concernant l'obésité, la position de sommeil ou l'hygiène du sommeil.

10. Système selon l'une quelconque des revendications 1 à 9, dans lequel le système de commande est en outre configuré pour exécuter les instructions lisibles par machine pour estimer une ou plusieurs caractéristiques physiques de l'obstruction physique, optionnellement, dans lequel les une ou plusieurs caractéristiques physiques incluent au moins une caractéristique physique sélectionnée parmi un groupe constitué d'une taille, d'une forme ou d'un emplacement de l'obstruction physique ; et/ou
dans lequel les une ou plusieurs caractéristiques physiques sont estimées sur la base d'une variation d'impédance acoustique ; et/ou
dans lequel les une ou plusieurs caractéristiques physiques sont estimées sur la base de l'analyse de la réflexion acoustique qui se produit à partir de l'obstruction physique dans les voies respiratoires de l'utilisateur ;
dans lequel les une ou plusieurs caractéristiques physiques sont estimées sur la base d'une comparaison entre (a) un ou plusieurs signaux acoustiques réfléchis en un point M avant l'entrée dans les voies respiratoires de l'utilisateur et (b) un ou plusieurs signaux acoustiques réfléchis en un point P après l'entrée dans les voies respiratoires de l'utilisateur ; et/ou
dans lequel les une ou plusieurs caractéristiques physiques sont estimées sur la base d'une distance parcourue par un ou plusieurs signaux acoustiques émis dans les voies respiratoires de l'utilisateur au-delà d'un point d'entrée, dans lequel le point d'entrée est une zone de la bouche ou une zone du nez de l'utilisateur, les voies respiratoires se trouvant dans une zone de la gorge de l'utilisateur.

11. Système selon l'une quelconque des revendications 1 à 10, dans lequel la caractérisation est en outre basée sur une mesure physique d'au moins une parmi la zone de la bouche, la zone du nez et la zone de la gorge ; et/ou
dans lequel les une ou plusieurs caractéristiques physiques sont estimées sur la base d'une morphologie de signal de la réflexion acoustique, optionnellement, dans lequel la morphologie de signal inclut une forme acoustique d'une onde acoustique de la réflexion acoustique ; et/ou
dans lequel les une ou plusieurs caractéristiques physiques sont estimées sur la base en partie d'une ou plusieurs caractéristiques physiques ou démographiques de l'utilisateur, optionnellement, dans lequel les une ou plusieurs caractéristiques de l'utilisateur sont sélectionnées parmi un groupe comprenant ou consistant en l'âge, le sexe, le poids, la taille de la bouche, la taille du cou ou un type d'interface utilisateur actuellement utilisé par l'utilisateur, ou toute combinaison de ceux-ci ; et/ou
dans lequel le système de commande est en outre configuré pour exécuter les instructions lisibles par machine pour identifier un emplacement d'un organe du corps de l'utilisateur, optionnellement, dans lequel l'organe du corps est un poumon ; et/ou
dans lequel le dispositif acoustique inclut un moteur, dans lequel le moteur est configuré pour émettre le signal acoustique ; et/ou
dans lequel le dispositif acoustique inclut un haut-parleur, le haut-parleur émettant le signal acoustique, optionnellement, dans lequel le haut-parleur génère le signal acoustique sous la forme d'un son, le son étant sélectionné parmi un groupe comprenant ou consistant en un ou plusieurs parmi un son standard, un son personnalisé, une fréquence inaudible, un bruit blanc, une impulsion à large bande, une forme d'onde sinusoïdale continue ou toute combinaison de ceux-ci ; et/ou
dans lequel le signal acoustique se présente sous la forme d'un son sélectionné parmi un groupe consistant en un son audible et un son ultrasonique ; et/ou
dans lequel le système de commande est en outre configuré pour exécuter les instructions lisibles par machine pour obtenir un cepstre de la réflexion acoustique, optionnellement, dans lequel le cepstre identifie une distance associée à la réflexion acoustique, la distance indiquant un emplacement de l'obstruction physique dans les voies respiratoires de l'utilisateur, optionnellement en outre, dans lequel le cepstre identifie une distance entre une interface utilisateur positionnée sur le visage de l'utilisateur et l'obstruction physique.

12. Système selon l'une quelconque des revendications 1 à 11, dans lequel la position de l'utilisateur inclut un ou plusieurs parmi un emplacement de l'utilisateur et une orientation de l'utilisateur ; et/ou
dans lequel le capteur de mouvement est sélectionné parmi un groupe consistant en une caméra, un capteur de lit, un accéléromètre, un capteur sonar et un capteur de radiofréquence tel qu'un capteur à bande hertzienne ultra-large à impulsions (IR-UWB).

13. Système selon l'une quelconque des revendications 1 à 12, comprenant en outre un système de thérapie respiratoire pour le traitement de l'apnée obstructive du sommeil, optionnellement dans lequel le système de thérapie respiratoire est capable de déterminer de manière indépendante une occurrence de l'événement d'apnée ou de l'événement d'hypopnée sur la base (i) de données de débit, (ii) de données de pression ou (iii) des deux (i) et (ii), détectées par le système de thérapie respiratoire ; et/ou
dans lequel le dispositif acoustique inclut un microphone pour détecter la réflexion acoustique du signal acoustique.

14. Système selon la revendication 1, comprenant en outre :
un système de thérapie respiratoire ;
le dispositif acoustique étant associé au système de thérapie respiratoire ; et
le système de commande étant en outre configuré pour exécuter les instructions lisibles par machine pour :
fournir de l'air sous pression, via le système de thérapie respiratoire, aux voies respiratoires de l'utilisateur pendant une session de sommeil ;
recevoir des données de débit et/ou des données de pression associées à l'air sous pression ; et déterminer, sur la base des données de débit et/ou des données de pression, une occurrence de l'événement d'apnée ou de l'événement d'hypopnée ;
dans lequel, optionnellement, le système de thérapie respiratoire est un système de pression positive des voies respiratoires (PAP).
